# EUROPEAN PATENT SPECIFICATION

(11) **EP 1 282 414 B1**
(45) Date of publication and mention of the grant of the patent: **05.01.2005**
(21) Application number: 01935095.8
(22) Date of filing: 04.05.2001
(51) Int. Cl.: A61K 31/27, A61P 43/00

(54) **FELBAMATE DERIVED COMPOUNDS FOR TREATING NEUROPATHIC PAIN**
FELABAMAT-DERIVATE ZUR BEHANDLUNG VON NEUROPATHISCHEM SCHMERZ
COMPOSES DERIVES DE FELBAMATE POUR LE TRAITEMENT DE LA DOULEUR NEUROPATHIQUE

(30) Priority: 05.05.2000 US 202145 P
(43) Date of publication of application: 12.02.2003
(73) Proprietor: University of Virginia Patent Foundation, Charlottesville, VA 22903-2442 (US)
(72) Inventor: MACDONALD, Timothy, L., Charlottesville, VA 22903 (US)
(74) Representative: Lucas, Brian Ronald
(86) International application number: PCT/US2001/014594
(87) International publication number: WO 2001/085149

(56) References cited:
- WO-A-00/47202
- WO-A-97/37652

## Description

### Background of the Invention

Felbamate (2-phenyl-1,3-propanediol dicarbamate) is a known pharmaceutical compound having been described in U.S. Pat. Nos. 2,884,444 and 4,868,327, the disclosures of which are expressly incorporated herein. Felbamate is a modulator of NMDA (N-methyl-D-aspartate) receptor function, and a glycine site antagonist but also has other reported mechanisms of actions.

Felbamate has also been reported to interact at the AMPA/kainate receptor, facilitate the function of the GABA receptor, and modulate Na.sup.+ channel conductance. Felbamate has also been demonstrated to decrease delayed neuronal cell death after kainic acid induced status epilepticus in animals. Glycine or d-serine were able to functionally reverse the anticonvulsant and ischemic protective effect of felbamate.

Felbamate has been proposed for use in treating various neurological disorders including the control of epileptic seizures. For example, U.S. Pat No. 4,978,680 discloses the use of felbamate for the prevention and control of epileptic seizures; U.S. Pat. No. 5,082,861 relates to_the use of felbamate for the prevention and control of epileptic seizures associated with complex partial seizures; and U.S. Pat. No. 5,292,772 relates to the use of felbamate for the prevention and control of epileptic seizures associated with Lennox-Gastaut syndrome. The disclosures of U.S. Pat. Nos. 4,978,680, 5,082,861 and 5,292,772 are expressly incorporated herein.

Felbamate has also been reported to have efficacy in reducing cellular damage resulting from vascular reperfusion (US Patent No. 5,462,966) and preventing and treating tissue damage resulting from an ischemic event (US Patent No. 5,055,489). For example, compositions comprising felbamate can be administered to control or prevent hypoxic damage resulting form stroke and other cerebral ischemic events. The disclosure of U.S. Pat. Nos. 5,462,966 and 5,055,489 are also expressly incorporated herein.

Felbamate was approved in July 1993 for the treatment of several forms of epilepsy. Felbamate demonstrated an excellent therapeutic index throughout preclinical and clinical trials with only relatively mild side effects observed and/or reported. In its first year of approval, between 100,000 and 125,000 patients were placed on felbamate therapy in the U.S. However, within the first year of felbamate's wide spread use, adverse reactions were reported, notably aplastic anemia and hepatotoxicity. (See Pennell et al., *Neurology.* **45**,456-460 (1995) and O'Neil et al., *Neurology*. **46,** 1457-1459 (1996)). The severity and frequency of occurrence of these side effects prompted a recommendation by the FDA in August 1994 to withdraw patients from felbamate therapy, unless the benefit of seizure control outweighed the risk of the reported toxicities.

### Summary of the Invention

The presently disclosed derivatives of felbamate have been shown to have activity as neuroprotectants and are believed to have biological activities similar to those of the parent felbamate compound. However, the present compounds have been modified to prevent the formation of metabolites that are believed to cause the adverse reactions associated with the use of felbamate. Accordingly, it is anticipated that the felbamate derivatives of the present invention can be substituted for felbamate for all the therapeutic uses that have been proposed for felbamate. In addition, many of the derivatives have enhanced activities allowing for the administration of lower therapeutically effective dosage forms.

The invention provides method for treating a patient suffering from neuropathic pain, comprising administering to the patient an effective amount of a compound of formula (I), (II), or (III): 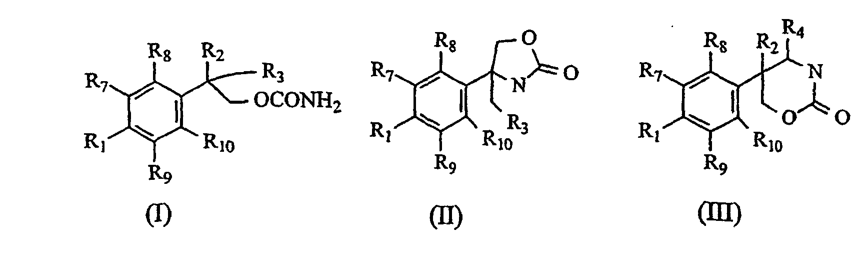 wherein:
R₁, R₇, R₈, R₉ and R₁₀ are each independently H, halo, alkyl, haloalkyl, NR₅R₆, hydroxy, or alkoxy;
R₂ is halo;
R₃ is hydroxy or -OCONH₂;
R₄ is hydroxy or carbonyl (=O); and
R₅ and R₆ are each independently alkyl (preferably, C₁-C₄alkyl);
or a pharmaceutically acceptable salt thereof.

The invention also provides a method for treating a patient suffering from obesity, comprising administering to the patient an effective amount of a compound of formula (I), (II), or (III), or a pharmaceutically acceptable salt thereof.

The invention also provides a method for treating glaucoma, comprising administering to the patient an effective amount of a compound of formula (I), (II), or (III), or a pharmaceutically acceptable salt thereof.

The invention also provides a method for treating a patient suffering from depression comprising administering to the patient an effective amount of a compound of formula (I), (II), or (III), or a pharmaceutically acceptable salt thereof.

The invention also provides a method for treating a patient suffering from a mood disorder comprising administering to the patient an effective amount of a compound of formula (I), (II), or (III), or a pharmaceutically acceptable salt thereof.

The invention also provides a method for treating a patient suffering from diabetic neuropathy comprising administering to the patient an effective amount of a compound of formula (I), (II), or (III), or a pharmaceutically acceptable salt thereof.

The invention also provides a method for treating a patient suffering from Alzheimer's disease, Parkinson's disease, or HIV dementia comprising administering to the patient an effective amount of a compound of formula (I), (II), or (III), or a pharmaceutically acceptable salt thereof.

The invention also provides a method for treating a patient suffering from Sepsis, Meningitis, CNS Vasculitis, Adrenoleukodystrophy, Impotence, Schizophrenia, Drug Addiction, Multiple Sclerosis, Fatigue, Lead Poisoning, Mitochondrial Myopathies, HIV Dementia, Amyotrophic Lateral Sclerosis, Attention Deficit Disorder, Narcolepsy, Childbirth Complications, Surgical Anesthesia, Traumatic Head and Spinal Cord Injury, Hypoglycemia, Tourette's Syndrome or Hepatic Encephalopathy comprising administering to the patient an effective amount of a compound of formula (I), (II), or (III), or a pharmaceutically acceptable salt thereof.

The invention also provides the use of a compound of formula (I), (II), or (III), or a pharmaceutically acceptable salt thereof to prepare a medicament useful for treating neuropathic pain in a mammal.

The invention also provides the use of a compound of formula (I), (II), or (III), or a pharmaceutically acceptable salt thereof to prepare a medicament useful for treating obesity in a mammal.

The invention also provides the use of a compound of formula (I), (II), or (III), or a pharmaceutically acceptable salt thereof to prepare a medicament useful for treating glaucoma in a mammal.

The invention also provides the use of a compound of formula (I), (II), or (III), or a pharmaceutically acceptable salt thereof to prepare a medicament useful for treating depression in a mammal.

The invention also provides the use of a compound of formula (I), (II), or (III), or a pharmaceutically acceptable salt thereof to prepare a medicament useful for treating a mood disorder in a mammal.

The invention also provides the use of a compound of formula (I), (II), or (III), or a pharmaceutically acceptable salt thereof to prepare a medicament useful for treating diabetic neuropathy in a mammal.

The invention also provides the use of a compound of formula (I), (II), or (III), or a pharmaceutically acceptable salt thereof to prepare a medicament useful for treating Alzheimer's disease, Parkinson's disease, or HIV dementia in a mammal.

The invention also provides the use of a compound of formula (I), (II), or (III), or a pharmaceutically acceptable salt thereof to prepare a medicament useful for treating Sepsis, Meningitis, CNS Vasculitis, Adrenoleukodystrophy, Impotence, Schizophrenia, Drug Addiction, Multiple Sclerosis, Fatigue, Lead Poisoning, Mitochondrial Myopathies, HIV Dementia, Amyotrophic Lateral Sclerosis, Attention Deficit Disorder, Narcolepsy, Childbirth Complications, Surgical Anesthesia, Traumatic Head and Spinal Cord Injury, Hypoglycemia, Tourette's Syndrome or Hepatic Encephalopathy, in a mammal.

A preferred compound useful for administration according to the methods of the invention is a compound of formula (I), (II), or (III): 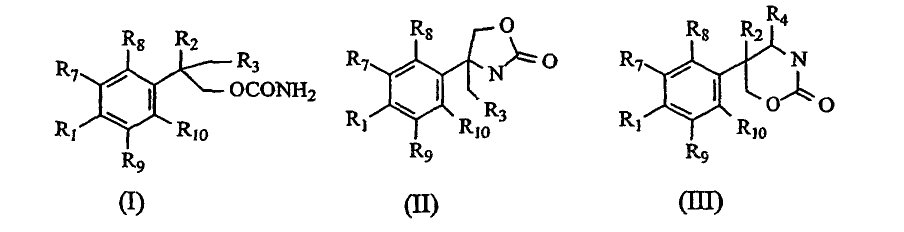 wherein:
R₁, R₇ and R₈ are each independently H, halo, alkyl, haloalkyl or hydroxy;
R₂ is fluoro;
R₃ is hydroxy or -OCONH₂;
R₄ is hydroxy or carbonyl (=O); and
R₉ and R₁₀ are each H;
or a pharmaceutically acceptable salt thereof.

The invention also provides novel compounds of formula (I), (II), or (III) disclosed herein, and salts thereof; as well as synthetic processes and intermediates useful for preparing compounds of formula (I), (II), or (III), and salts thereof.

### Detailed Description of the Invention

In describing and claiming the invention, the following terminology will be used in accordance with the definitions set forth below.

As used herein, the term "pharmaceutically acceptable carrier" encompasses any of the standard pharmaceutical carriers, such as a phosphate buffered saline solution, water and emulsions such as an oil/water or water/oil emulsion, and various types of wetting agents.

As used herein, "effective amount" means an amount sufficient to produce a selected effect. For example, an effective amount of a felbamate derivative for treating neuropathic pain is an amount sufficient to reduce the incidence or severity of such pain.

The general chemical terms used in the description of the compounds of the present invention have their usual meanings. For example, the term "alkyl" by itself or as part of another substituent means a straight or branched aliphatic chain having the stated number of carbon atoms. Preferably, alkyl is C₁-C₆alkyl, and more preferably C₁-C₄alkyl (e.g. methyl, ethyl, propyl, isopropyl, or butyl).

The term "halo" includes bromo, chloro, fluoro, and iodo.

The term, "parenteral" means not through the alimentary canal but by some other route such as subcutaneous, intramuscular, intraspinal, or intravenous.

As used herein, the term "treating" includes alleviating the symptoms associated with a specific disorder or condition and/or preventing or eliminating said symptoms.

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

The following metabolic pathway (Scheme I) of felbamate (1) has been proposed that leads to the reactive metabolite, 3-carbamoyl-2-phenylpropionaldehyde (3).

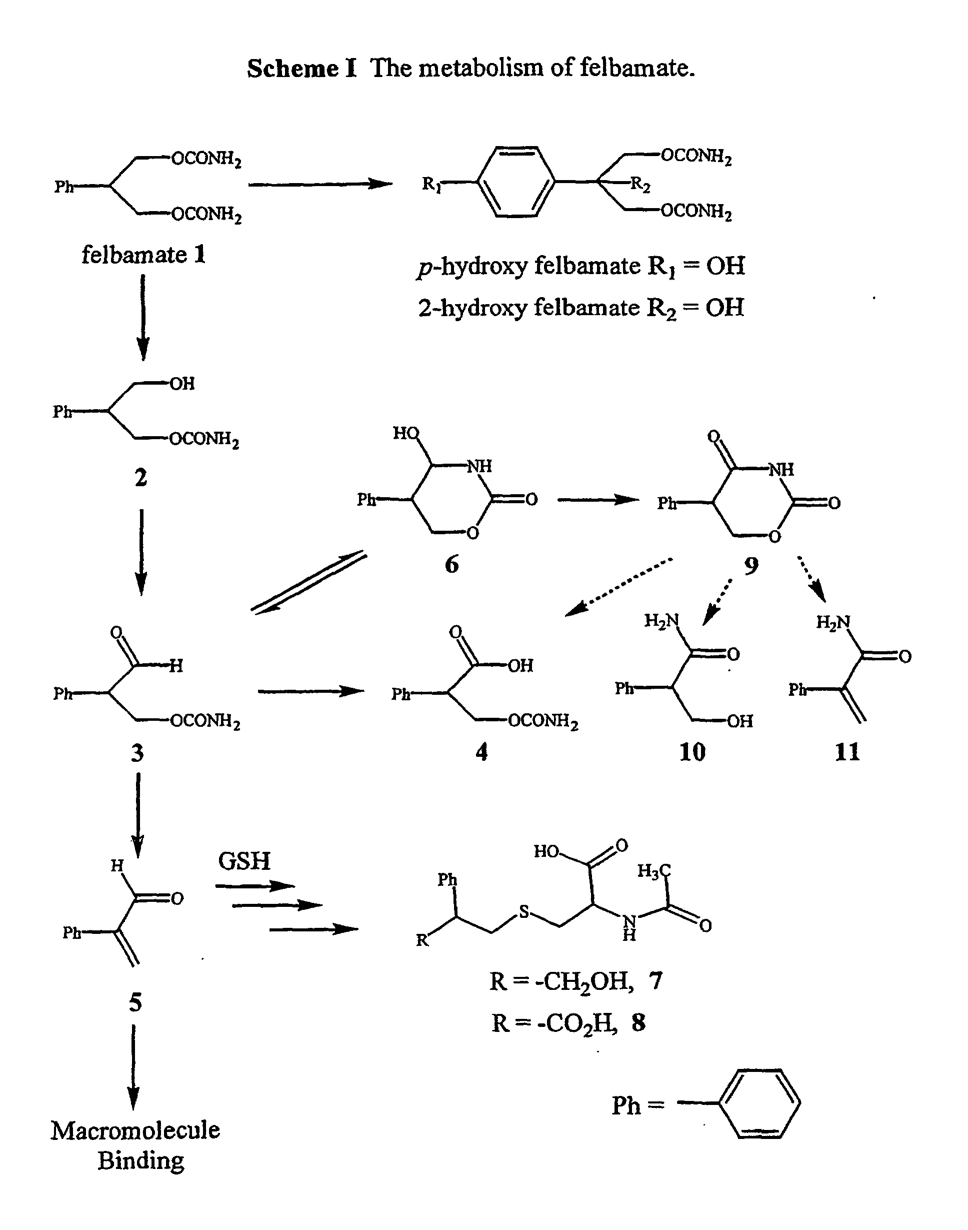

3-Carbamoyl-2-phenylpropionaldehyde (3) is believed to be a reactive intermediate in the oxidation of 2-phenyl-1,3-propanediol monocarbamate **(2)** to the major human metabolite 3-carbamoyl-2-phenylpropionic acid **(4)**. In addition, the aldehyde carbamate **(3)** was found to undergo spontaneous elimination to form the α,β unsaturated aldehyde, 2-phenylpropenal **(5)**, commonly known as atropaldehyde. Atropaldehyde has been proposed to play a role in the development of toxicity during felbamate therapy.

Evidence for atropaldehyde formation *in vivo* has been reported with the identification of modified N-acetyl-cysteine conjugates **7** and **8** of atropaldehyde in both human and rat urine after felbamate administration. Identification of the atropaldehyde derived mercapturic acids in urine after felbamate administration is consistent with the hypothesis that atropaldehyde is formed *in vivo* and that it reacts with thiol nucleophiles.

Based on the hypothesis that the toxicity associated with felbamate administration is directly correlated to the amount of atropaldehyde formed, the present invention is directed to the development of a new class of agents structurally related to felbamate that cannot undergo metabolism to atropaldehyde.

In accordance with the present invention the benzylic hydrogen of felbamate (1) is replaced with a substituent "R₂" as shown in the following metabolic scheme (Scheme II). R₂ is halo, and in one preferred embodiment the substituent is a fluorine atom.

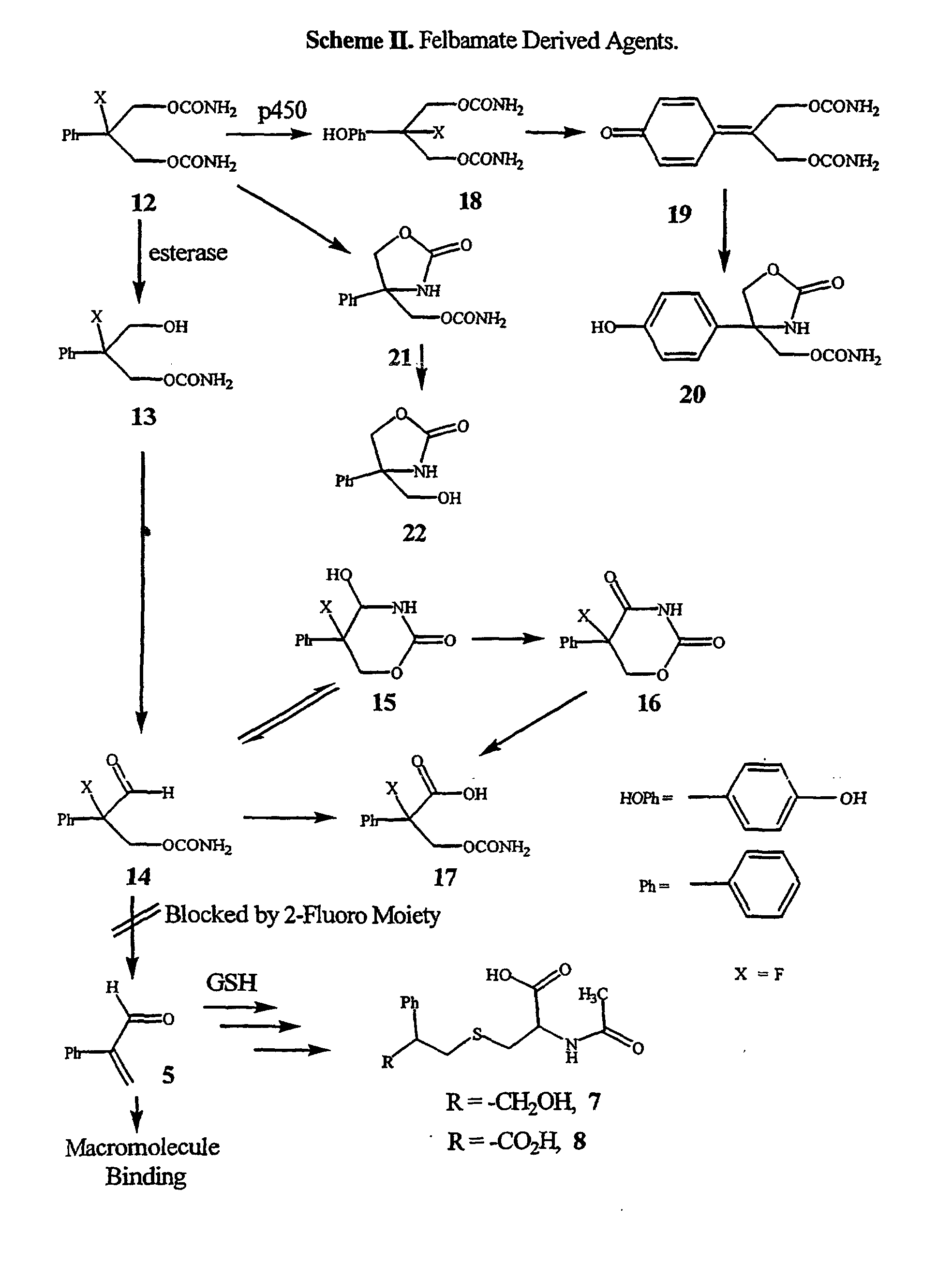

2-Fluoro-2-phenyl-1, 3-propanediol dicarbamate (Fluorofelbamate; 12) and 2-Fluoro-2-phenyl-1, 3-propanediol monocarbamate fluoro monocarbamate felbamate (13) are derivatives of known antiepileptic agents. These agents represent a new class of anti-epileptics that, while possessing structural similarity to felbamate, will not exhibit felbamate's metabolic profile and will not induce adverse reactions such as those associated with the use of felbamate.

In accordance with one embodiment of the present invention these compounds can be further modified to include substituents at the para position of the benzene ring of the felbamate derivative to preclude the formation of other potentially toxic species via the cytochrome P450 pathway. This potential cytochrome P450 mediated toxicity may be associated with the 2-substituted-2-phenyl-1,3-propanedioldicarbamate derivatives of the present invention by formation of electrophilic species such as 19, which may also be formed from dihydroxy felbmate, a known metabolite of felbamate. Febamate derivative compounds that are substituted at the para position are blocked from being metabolized by certain members of the cytochrome P450 metabolic family. For example, two preferred compounds are diflurofelbamate (**23**) and difluoro monocarbamate felbamate (**24**). These febamate derivatives are incapable of being metabolized to atropaldehyde and also are blocked from being metabolized by certain members of the cytochrome P450 metabolic family.

Parafluoro Substituted Derivatives of Felbamate

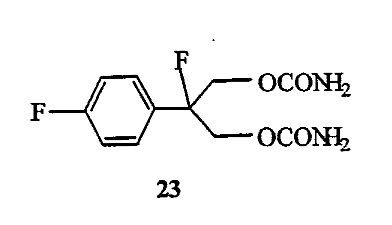

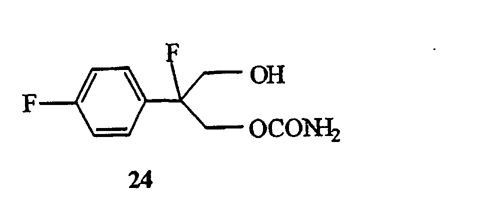

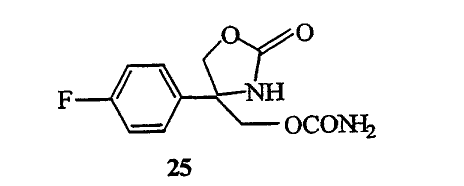

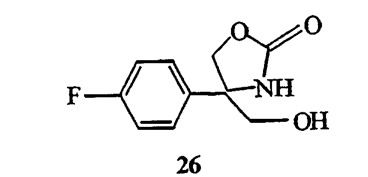

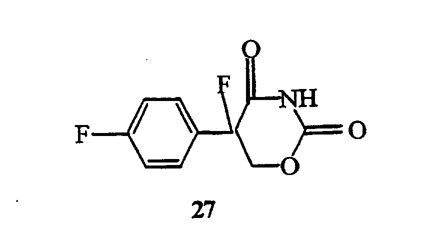

The present invention also encompasses the derivatives of the potentially active metabolites of felbamate. In particular this includes fluoro oxazinane-dione **16** and difluoro oxazinane-dione, which are derivatives of the felbamate metabolite oxazinane-dione **9**. The structure of oxazinane-dione **9** bears intriguing similarity to several established anti-epileptic drugs including phenobarbital, phenytoin, oxazinane-dione, metharbital and ethotoin, as illustrated below:

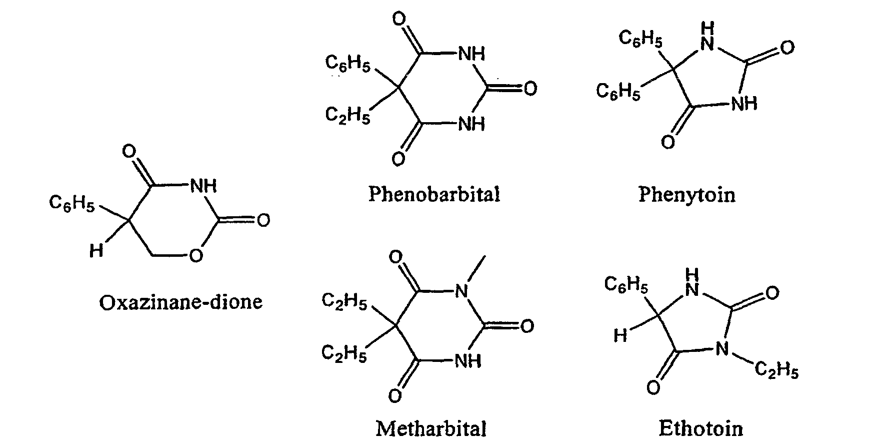

It is anticipated, therefore, that the oxazinane-dione **9** is responsible for some aspects of felbamate's efficacy *in vivo*. As patients undergoing felbamate therapy for seizure control ingest large quantities of felbamate (grams per day), even a 1 - 2% conversion to a pharmacologically active metabolite could have significant effects. This could play an important role in the seizure control observed with felbamate, particularly if the metabolite (i.e., the oxazinane-dione) was a more potent compound. In light of the possibility that the oxazinane-dione 9 could be a metabolic precursor to the major human metabolite, acid carbamate 4, it may be formed in significant quantities (the acid carbamate was reported to represent ∼ 12% of a dose). Because the parent oxazinane-dione **9** has been found to be unstable at relevant pH, oxazinane-diones 16 are considered candidates for development as potential antiepileptic agents. Also included in this class are cyclic congeners **21** and **22**, which may be formed from felbamate derivatives (see scheme II). Also included is **20,** which may be an unknown metabolite of felbamate **1** and could be produced from felbamate derivative **12** by the cytochrome P450 metabolism.

A preferred compound for administration according to the methods of the invention is a compound having the general structure: 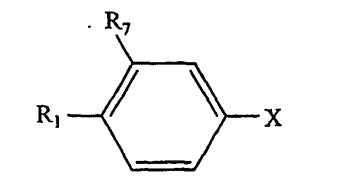 wherein X is selected from the group consisting of 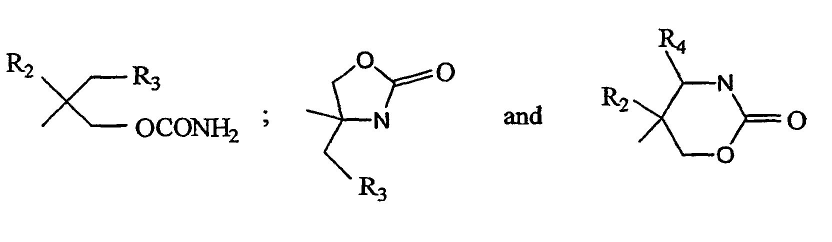 and R₁, and R₇ are independently selected from the group consisting ofH, halo, alkyl, haloalkyl, -NR₅R₆, hydroxy, and alkoxy, R₂ is Cl or F, R₃ is hydroxy or -OCONH₂, R₄ is hydroxy or carbonyl, R₅ and R₆ are independently C₁-C₄ alkyl.

Another preferred compound for administration according to the methods of the invention is a compound having the general structure: 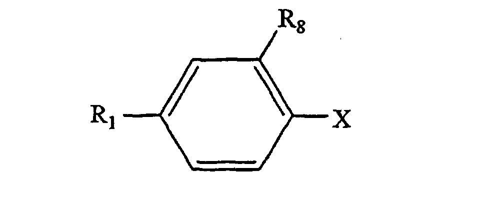 wherein X is selected from the group consisting of 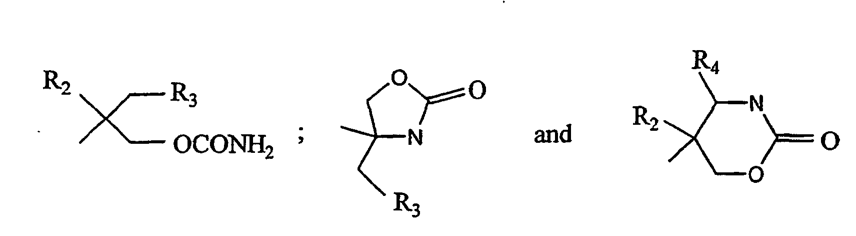 and R₁, and R₈ are independently selected from the group consisting ofH, halo, alkyl, haloalkyl, -NR₅R₆, hydroxy, and alkoxy, R₂ is Cl or F, R₃ is hydroxy or -OCONH₂, R₄ is hydroxy or carbonyl, R₅ and R₆ are independently C₁-C₄ alkyl.

Another preferred compound for administration according to the methods of the invention is a compound is selected from the group consisting of 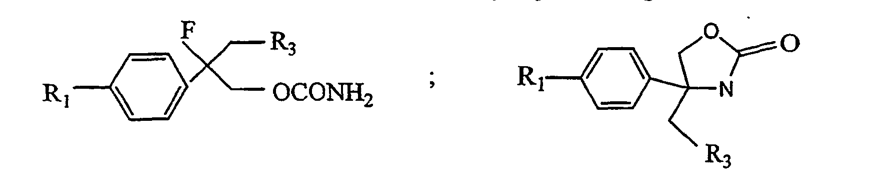 and 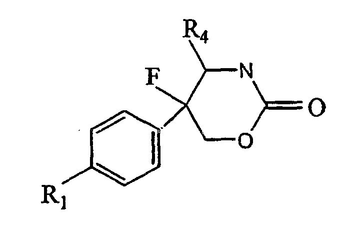 wherein R₁ is selected from the group consisting ofH, halo, alkyl, haloalkyl, -NR₅R₆, hydroxy, and alkoxy, R₃ is hydroxy or -OCONH₂, R₄ is hydroxy or carbonyl, and R₅ and R₆ are independently C₁ -C₄ alkyl.

Another preferred compound for administration according to the methods of the invention is a compound is selected from the group consisting of 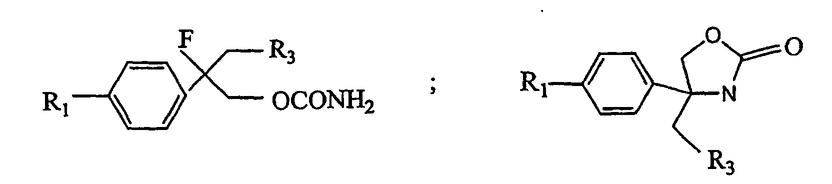 and 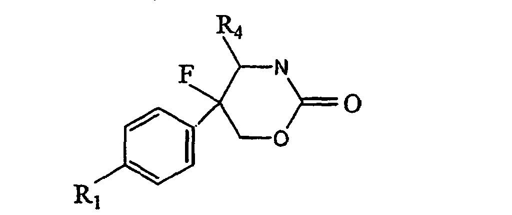 wherein R₁ is selected from the group consisting of H or halo, R₃ is hydroxy or -OCONH₂, and R₄ is hydroxy or carbonyl.

Another more preferred compound for administration according to the methods of the invention is a compound selected from the group consisting of: 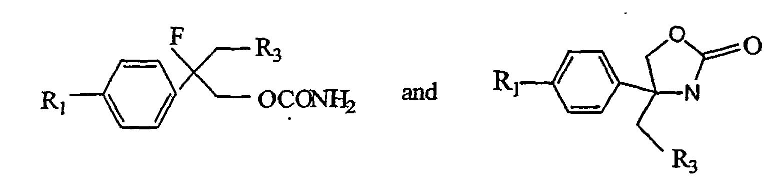 wherein R₁ is selected from the group consisting of H, F, Cl, CF₃ and hydroxy and R₃ is hydroxy or -OCONH₂. In one preferred embodiment R₁ is H or F and R₃ is -OCONH₂.

Compositions comprising the felbamate derivative of the present invention can be used to treat patients suffering from a neurological diseases such as epileptic seizures or can be used to prevent or treat reperfusion injuries resulting from stroke, myocardial infarction, and spinal chord perfusion-type injuries. It is believed that the felbamate derivatives of the present invention also have utility for treating conditions characterized by the presence of reactive oxygen species (ROS).

The felbamate derivative of the present invention can be combined with pharmaceutically acceptable carriers, stabilizing agents, solubilizing agents, and fillers known to those skilled in the art for administration to the patient. The compositions can be formulated using standard delivery vehicles and standard formulations for oral, parenteral or transdermal delivery.

Another preferred compound for administration according to the methods of the invention is a compound selected from the group consisting of: 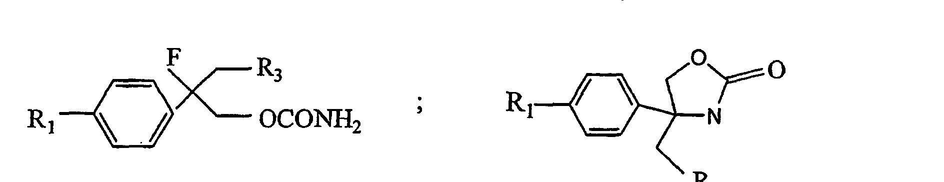 and 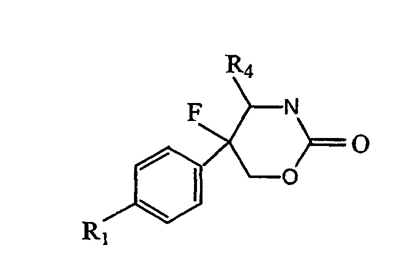 wherein R₁ is selected from the group consisting ofH, halo, alkyl, haloalkyl and hydroxy, R₃ is hydroxy or -OCONH₂, and R₄ is hydroxy or carbonyl, in combination with a pharmaceutically acceptable carrier.

Another more preferred compound for administration according to the methods of the invention is a compound selected from the group consisting of : 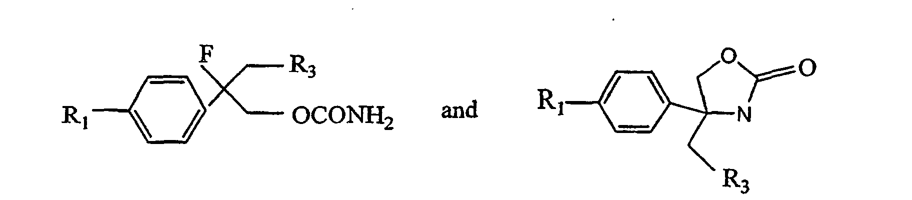 wherein R₁ is selected from the group consisting of H, F, Cl, CF₃ and hydroxy, R₃ is hydroxy or -OCONH₂ in combination with a pharmaceutically acceptable carrier.

It is anticipated that the present felbamate derivatives will have similar activity to felbamate and therefore the present derivatives are anticipated to be efficacious in treating the disorders where felbamate is therapeutically effective. More particularly, US Patent Nos. 5,942,540 and 5,728,728, the disclosures of which are expressly incorporated herein, describe a number of diseases or conditions that can be treated by the administration of felbamate. The felbamate derivatives of the present invention are also useful in treating these diseases.

Obesity is a common human disorder which affects 10-15% of the population, of which up to 5% may be severely obese. It is estimated that the mortality from obesity is between 300,000 to 400,000 per annum. Obesity is commonly measured by the BMI (body mass index) which is the weight in kilograms divided by the height in meters squared. The degree of obesity is determined by comparisons against standard deviations above the means for males and females.

The etiology of obesity is unknown but occurs when energy intake exceeds energy expenditure. Appetite is controlled by the ventromedial hypothalamus and complex interconnections with the limbic system and other portions of the brain. Recent neurochemical research has implicated leptin, GLP-1 (glucagon-like peptide 1) and neuropeptide-Y in the control of appetite. Leptin is a natural appetite suppressant which is released from adipose cells, travels to the brain and appears to exert some control over appetite and long term weight control. A defective leptin receptor has been postulated to be involved in obese patients. GLP-1, a brain hormone which promotes satiety, is believed to regulate short-term appetite. Neuropeptide-Y is a potent stimulator of appetite whose effects can be blocked by leptin and GLP-1. The complete interaction of these compounds remains to be elucidated.

Felbamate has been known to induce anorexia in patients treated with epilepsy. In a study of felbamate as add-on therapy in partial epilepsy in children, weight loss was transient and returned to normal after twenty weeks (Carmant J., Pediatr 125:481-486, 1994). Weight loss of 4-5% was noted in patients on felbamate monotherapy (Faught E., Neurology 43:688-692, 1993). It has been suggest that weight loss from felbamate is due to NMDA receptor modulation in the hypothalamic structures involved in appetite control.

Felbamate, administered chronically to humans in oral doses of from about 100-15,000 mg/day, advantageously from about 1200-7200 mg/day (serum levels ranging from about 25-300 ug/ml), is efficacious in producing weight loss in obesity, type-II diabetes, and other genetic obesity disorders. It is anticipated that administration of the felbamate derivatives of the present invention art similar dosage ranges will similarly be efficacious in producing weight loss in individuals. Therefore, in accordance with one embodiment of the present invention the felbamate derivatives of the present invention are formulated as pharmaceutical compositions and administered to individuals to induce weight loss in those individuals.

Spasticity is a human motor disorder manifested by an increase in muscle tone and an exaggeration of deep tendon reflexes due to lesions of the corticospinal system. The spasticity is proportional to the rate and degree of stretch placed on the muscle. The most common causes are multiple sclerosis and spinal cord injury. Spasticity produces multiple medical complications, pain, and depression.

The etiology of spasticity is a decrease or malfunctioning of inhibitory mechanisms in the spinal cord leading to hyper-excitability of the tonic stretch and other reflexes. The mechanism may involve a decrease in both presynaptic GABA-ergic inhibition and postsynaptic inhibition. Noradrenergic receptors become supersensitive distal to spinal cord injury which is the rationale for using alpha-2 agonists as a treatment in spinal cord injury. GABA and glycine are the main inhibitory neurotransmitters in the spinal cord. Glycine acts at both the strychnine-insensitive and strychnine-sensitive receptors, the latter being more common in the spinal cord.

The pharmacotherapy of spasticity is directed at the potentiation of inhibitory transmission within the spinal cord, such as the mediation of presynaptic inhibition by GABA. Excitatory amino acids (EAA) increase spasticity and non-competitive NMDA antagonists depress spinal polysynaptic reflexes by inhibiting the release of EAA (Schwarz M., In Thilman AF, Ed. Spasticity, pp 85-97, 1993). Felbamate has both GABA enhancing properties and glycine-site strychnine insensitive antagonist properties which suggests efficacy in the treatment of spasticity.

Felbamate, administered chronically in oral doses of from about 100-15,000 mg/day, advantageously from about 1200-7200 mg/day (serum levels ranging from 25-300 ug/ml), is efficacious in reducing spasticity from both supraspinal and spinal lesions. Accordingly it is anticipated that administration of the felbamate derivatives of the present invention art similar dosage ranges will also alleviate spasticity from both supraspinal and spinal lesions.

Depression or mood disorders are psychopathologic states in which a disturbance of mood is either a primary determinant or constitutes the core manifestation. Secondary depression is an affective disorder caused by a systemic or neurological disease. Examples of neurologic diseases include but are not limited to multiple sclerosis, Parkinson's disease, head trauma, cerebral tumors, post-stroke, early dementing illness, and sleep apnea, while systemic diseases include infections, endocrine disorders, collagen vascular diseases, nutritional deficiencies and neoplastic disease. Secondary depression is common in post-myocardial infarct patients and carry a mortality three times that of non-depressed post-myocardial patients.

Felbamate, administered chronically in oral doses of from about 100-15,000 mg/day, advantageously from about 1200-7200 mg/day (serum levels ranging from 25-300 ug/ml), is efficacious in reducing secondary symptomatic depression in both human systemic and neurologic diseases. Accordingly it is anticipated that administration of the felbamate derivatives of the present invention art similar dosage ranges will also alleviate secondary symptomatic depression in both human systemic and neurologic diseases.

In accordance with one embodiment the felbamate derivatives of the present invention are used to alleviate pain and in particular pain of neuropathic origin. Neuropathic pain is a chronic condition in which NMDA receptors in neural pain pathways have become "kindled" to an abnormally high level of sensitivity so that they spontaneously convey nerve messages that the patient perceives as pain even though no painful stimulus has been inflicted (see US Patent No. 5,925,634, the disclosure of which is incorporated herein). Excessive activation of NMDA receptors is believed responsible for the generation of "neuropathic" pain, a type of pain which is sometimes called "neurogenic pain" or "wind-up" pain (Woolf et al 1989; Kristensen et al 1992; Yamamoto and Yaksh 1992).

By mechanisms that are poorly understood, pathological changes associated with diabetes are conducive to the generation of neuropathic pain, a condition known as "diabetic neuropathy". One of the distinguishing characteristics of neuropathic pain is that morphine and related pain-killing drugs which are effective in controlling other types of pain are usually ineffective in controlling neuropathic pain (Backonja 1994). NMDA receptors are found in the pain-transmitting structures of the spinal cord, thalamus and certain layers of the cerebral cortex and several recent reports indicate that NMDA antagonists can prevent or ameliorate neuropathic pain (Davar et al 1991; Mao et al 1992; Seltzer et al 1991; Neugebauer et al 1993; Kristensen et al 1992; Backonja et al 1994).

For example, intractable tic douloureux (Chesire, W. P., Clin. J. Pain, 11:139-142, 1995), has been effectively treated with felbamate at doses of 1200-2400 mg/day. Conditions such as peripheral neuropathy, terminal cancer pain and failed back surgery which are intractable to current treatment modalities also benefit from felbamate treatment. In the formalin injection animal pain model, glycine site antagonists (Millan, M. J., Neurosci. Lett., 178(1):139-143, 1994, Vaccarino, A. L., Brain Res., 615(2):331-334, 1993) decreased the late phase pain response. In a rat model of painful peripheral neuropathy, felbamate produced significant reductions in all measures of pain (Imamura, I., J. Pharm. Exp. The., 275(1):177-182,1995). Specifically, the action of felbamate was antihyperalgesic and antiallodynic rather than analgesic.

The NMDA receptor antagonist felbamate derivatives of the present invention function similar to felbamate to block the NMDA receptor at the glycine site and thus these compounds will also prevent chronic pain transmission. Therefore, in accordance with one embodiment, the felbamate derivatives of the present invention are utilized to provide symptomatic relief from neuropathic pain without producing central nervous system side effects.

In accordance with one embodiment, the felbamate derivatives are administered chronically in oral doses ranging from 100-15,000 mg/day, and more preferable about 1200 to about 7200 mg/day (serum levels ranging from about 25 ug/ml to about 300 ug/ml), to alleviating chronic pain.

In accordance with one embodiment a method is provided for treating a patient suffering from neuropathic pain. The method comprises the step of administering a composition comprising a compound selected from the group consisting of 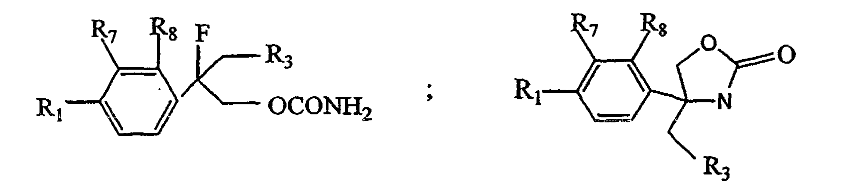 and 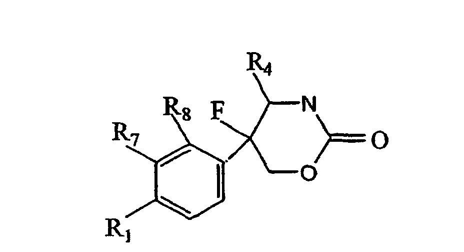 wherein R₁, R₇ and R₈ are independently selected from the group consisting of H, halo, alkyl, haloalkyl and hydroxy; R₃ is hydroxy or -OCONH₂; and R₄ is hydroxy or carbonyl. In accordance with one embodiment the felbamate derivative is a compound selected from the group consisting of: 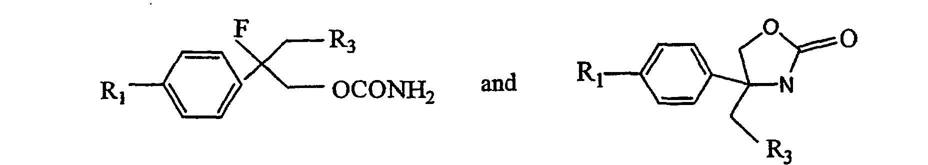 wherein R₁ is selected from the group consisting of H, F, Cl, CF₃ and hydroxy, R₃ is hydroxy or -OCONH₂. The felbamate derivatives can be combined with a pharmaceutically acceptable carrier for oral or parenteral administration for preventing and reducing neuropathic pain.

In accordance with one embodiment a method is provided for treating a patient suffering from glaucoma. In particular, it has been reported that NMDA receptor antagonists have efficacy in treating patients that do not respond to standard therapy. The method comprises the step of administering a composition comprising a compound selected from the group consisting of 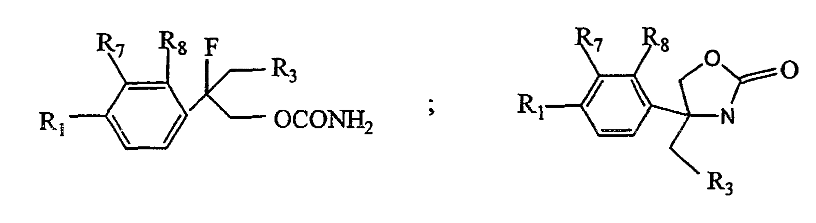 and 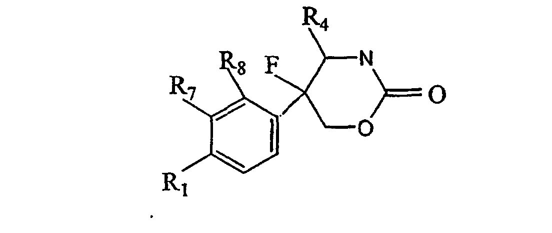 wherein R₁, R₇ and R₈ are independently selected from the group consisting of H, halo, alkyl, haloalkyl and hydroxy; R₃ is hydroxy or -OCONH₂; and R₄ is hydroxy or carbonyl. In accordance with one embodiment the felbamate derivative is a compound selected from the group consisting of 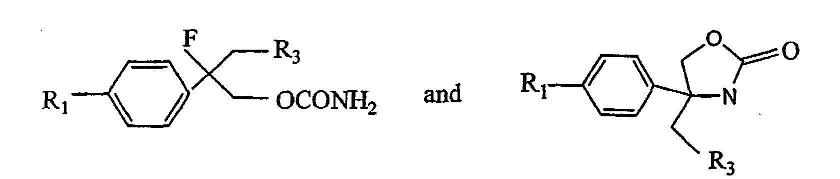 wherein R₁ is selected from the group consisting of H, F, Cl, CF₃ and hydroxy, R₃ is hydroxy or -OCONH₂. The felbamate derivatives can be combined with a pharmaceutically acceptable carrier for oral or parenteral administration for preventing and reducing neuropathic pain.

It has been reported that felbamate is effective in treating the following conditions/diseases associated with excessive activation of the NMDA receptor: Sepsis, Meningitis, CNS Vasculitis, Adrenoleukodystrophy, Impotence, Schizophrenia, Drug Addiction, Multiple Sclerosis, Fatigue (including fatigue associated with chronic diseases, such as multiple sclerosis, post-polio syndrome and Parkinson's, as well as chronic fatigue syndrome), Lead Poisoning, Mitochondrial Myopathies, HIV Dementia, Depression, Amyotrophic Lateral Sclerosis, Parkinson's Disease, Attention Deficit Disorder, Narcolepsy, Alzheimer's Disease, Childbirth Complications (i.e. premature labor, prolonged labor, hypoxia, etc. which place the fetus at risk for cerebral ischemic damage and cerebral palsy), Surgical Anesthesia (as a prophylactic treatment to reduce risk of brain damage from hypoxia, anoxia, cerebral embolism (i.e., fat, air), hypotension, hypoglycemia etc.), Traumatic Head and Spinal Cord Injury, Hypoglycemia, Tourette's Syndrome and Hepatic Encephalopathy, see US Patent No. 5,728,728, the disclosure of which is incorporated herein. Accordingly, it is anticipated that the NMDA receptor antagonists of the present invention can be used to treat these and other conditions that are associated with excessive activation of the NMDA receptor.

In one embodiment of the present invention a pharmaceutical composition comprising a felbamate derivative of the present invention is administered to a patient suffering from Alzheimer's Disease (DAT) to alleviate symptoms associated with the disease. Alzheimer's Disease (DAT) is a progressive dementing illness which is caused by an abnormal form of amyloid deposition in the brain. Excessive amyloid deposition induces glutamate toxicity of the NMDA receptor, resulting in neuronal death in areas of the brain that have a high density of NMDA receptors such as the hippocampus and cerebral cortex (areas of maximal neuronal death in DAT). The decrease in binding of NMDA receptors in DAT visual cortex is correlated with increased numbers of neurofibrillary tangles. (Carlson, M. D., Neurobiol. Aging, 14(4): 343-352,1993). Studies in animals have shown that glycine antagonist improve learning and attenuate scopalamine memory deficits (Fishkin, R. J., Behav. Neurol. Biol., 59(2):150-157, 1993, Finkelstein, J. E., Pharmacol. Biochem. Behav., 49(3):707-710, 1994, Baxter, M. G., Neurobiol. Aging, 15(2):207-213, 1994). Accordingly, the felbamate derivatives of the present invention, having glycine site NMDA antagonist with cognitive enhancement and neuronal protection properties, are useful for treating DAT and preventing DAT associated neuronal degeneration.

In another embodiment of the present invention a pharmaceutical composition comprising a felbamate derivative of the present invention is administered to treat a patient suffering from Parkinson's Disease. Parkinson's Disease (PD) is a selective degeneration of predominately D₂ dopaminergic neurons in the substantia nigra (motor portion of the basal ganglia) which produces progressive motor symptoms of rigidity and bradykinesia (slowness of movement). An NMDA-excitatory mechanism of early neuronal cell death is involved in the etiology of PD. Felbamate has been shown to antagonize the D₂ (dopamine receptor) in an animal model of cataplexy (Kretchmer, B. D., Neurosci Lett., 179(1-2):115-118, 1994). Accordingly, the felbamate derivatives of the present invention, having NMDA glycine site antagonist activity prevents NMDA receptors from being excessively stimulated, thus preventing progressive motor weakness and death (neuroprotection) in PD.

In another embodiment of the present invention a pharmaceutical composition comprising a felbamate derivative of the present invention is administered to treat a patient suffering from HIV Dementia. Patients who become HIV(+) have early pre-clinical brain neuronal deterioration as measured by NMR spectroscopy. When HIV(+) patients convert to AIDS, brain involvement produces dementia and is universally fatal. The mechanisms of brain deterioration appear to involve production of substances (i.e., quinolinic acid) that activate NMDA receptors and cause neuronal death by inducing intra cellular Ca.sup.++ overload. Accordingly, the felbamate derivatives of the present invention, having NMDA glycine site antagonist activity function as neuroprotection agents and prevent neuronal death.

When administered orally, the compounds of the present invention can be administered as a liquid solution, powder, tablet, capsule or lozenge. The felbamate derivative compounds can be used in combination with one or more conventional pharmaceutical additive or excipients used in the preparation of tablets, capsules, lozenges and other orally administrable forms. When administered as an intravenous solution, the derivatives of the present invention can be admixed with conventional IV solutions.

The compounds of the present invention are administered at a dose range effective to alleviate the symptoms associated with the disorder. In accordance with one embodiment the felbamate derivative (active agent) is administered in a dosage form of about 0.1 mg/kg to about 5.0 g/kg, and more particularly about 0.25 mg/kg to about 1 g/kg. The dosage will vary based on the route of administration and the condition/disorder to be treated.

### Example 1

### Use of an LC/MS method to quantify the two atropaldehyde derived mercaptuic acids 7 and 8 in a patient population treated with felbamate.

Although the FDA has recommended patients be given felbamate therapy only when other therapies have failed, it is estimated that 8,000-12,000 patients remain on felbamate therapy in the United States (12). While the mercapturic acids are generated from addition to glutathione, any similar nucleophile (i.e., nucleophilic amino acids of proteins or DNA bases) would be expected to undergo conjugation to atropaldehyde. The more atropaldehyde generated *in vivo* the greater the likelihood that a critical target will be alkylated, leading to toxicity. Therefore, it is reasonable to expect that the potential for toxicity will correlate to the amount of atropaldehyde formed. Further, the amount of atropaldehyde derived mercapturic acids excreted in urine will be a function of the amount of atropaldehyde formed.

In accordance with one aspect of the present invention an analytical method is described for quantifying the relevant metabolites excreted in patient urine as a measurement of a patient's susceptibility to toxicity associated with felbamate therapy. The method of determining a patient's susceptibility to adverse side affects from felbamate administration comprises the steps of obtaining a biological sample from the patient, preferably a urine sample, quantifying the mercapturic acid metabolites present in the sample and extrapolating the amount of atropaldehyde formed. In accordance with one embodiment standard liquid chromatography and mass spectroscopy (LC/MS) is used for quantifying the relevant metabolites excreted in patient urine.

### Materials and Methods

**Chemicals and Instruments.** All reagents were purchased from either Aldrich Chemical Co. or Sigma Chemical Co. and were of the highest quality available. HPLC was performed on a Waters 2690 Separations Module with a Waters 484 tunable absorbance detector (at 214 nm) using a Waters Symmetry C₁₈ (2.1 mm x 150 mm) column. Mass Spectra were obtained by coupling this LC system to a Finnigan MAT LCQ ion trap mass spectrometer equipped with an electrospray ionization source. NMR spectra were recorded on a General Electric QE300 spectrometer at 300 MHz and chemical shifts are reported in ppm. Melting points were determined on a Thomas-Hoover UNI-MELT apparatus and are uncorrected.

**Synthesis. 2-Phenyl-(1,1,3,3-tetra-deuterio )-1,3-propanediol.** 2- Phenyl-(1,1,3,3-tetra-deuterio )-1,3-propanediol was obtained by reduction of diethyl phenylmalonate with LiAlD₄ using methodology described previously for the formation of 2-phenyl-1,3-propanediol (1). The isotopic purity of the product was determined to be ≥98% as assessed by¹H NMR and GC/MS. (mp = 48 - 50 °C) ¹H NMR (CDCl₃): δ2.6 (s, 2H), 3.0 (s, 1H), 7.3 (m, 5H). ¹³C NMR (CDCl₃): δ49.8, 127.7, 128.5, 129.3,139.9. GC/MS (CI-methane) MH⁺ = 157- fragment ions: 139, 121, 106, 93. Elemental Analysis: theoretical C, 69.20; H, 7.74 found C, 68.98; H, 7.68. Molecular mass is calculated with 4 ²H atoms but the instrument used for elemental analysis observes each deuterium as though it were hydrogen. Therefore, the theoretical value for elemental analysis of hydrogen is determined based on the presence of 12 ¹H (i.e., 12 x 1.008 = 12.096/156.21 = 7.74%).

**2-Phenyl-(I,I,3,3-tetra-deuterio )-1,3-propanediol monocarbamate.** The d₄-monocarbamate alcohol was prepared from the d₄-diol using methodology described previously (1). The isotopic purity of the product was determined to be ≥98% as assessed by ¹H NMR and LC/MS. (mp = 71- 72°C) ¹H NMR ((CD₃)₂CO): δ3.1 (s,1H), 4.8 (bs, 2H), 7.3 (m, 5H). LC/ESI-MS: MH⁺ = 200.0.

**3-Carbamoyl-(3,3-di-deuterio )-2-phenylpropionic acid.** The d₂-acid carbamate was obtained essentially as described by Adusumalli *et al*. except that the d₄-monocarbamate alcohol was used as starting material (10). The isotopic purity of the product was determined to be ≥98% as assessed by ¹H NMR and LC/MS. (mp = 99-102 °C) ¹H NMR ((CD₃)₂SO): δ 3.9 (s, 1H), 5.8 (bs, 2H), 7.3 (m, 5H), 12.4 (bs, 1H). ¹³C NMR ((CD₃)₂CO): δ54.1, 127.3, 128.7, 128.7, 139.3, 155.6, 201. 7. LC/ESI-MS: MH⁺ = 211.9. Elemental Analysis: theoretical C, 56.87; H, 5.25; N, 6.63 found C, 56.74; H, 5.31; N, 6.57.

**2-Phenyl-(1,1,3,3-tetra-deuterio )-1,3-propanediol dicarbamate.** d₄-Felbamate was made using methodology described previously except that d₄-monocarbamate alcohol was used as starting material (2). The isotopic purity of the product was determined to be ≥ 98% as assessed by ¹H NMR and LC/MS. The spectral data obtained for this compound are in agreement with published values (11). (mp = 148- 150 °C) ¹H NMR ((CD₃)₂SO): δ 3.1 (s, 1H), 6.4 (bs, 4H), 7.2 (m, 5H). LC/ESI-MS: MH⁺ = 243.1. Elemental Analysis: theoretical C, 54.53; H, 5.83; N, 11.56 found C, 54.63; H, 5.84; N, 11.64.

**N-d**_{**3**}**-acetyl-L-cysteine**. Acetic anhydride (d₆) (0.29 g, 2.7mmol) was added to a solution of Cys(trt)-OH in 20 mL DMF and 1 mL pyridine and stirred overnight. The reaction mixture was diluted with 50 mL ether and extracted with saturated aqueous lithium bromide (2 x 50 mL). The organics were dried over sodium sulfate and the solvent was removed under reduced pressure. This intermediate was purified by flash chromatography using 1:1 methanol and chloroform affording a white solid: ¹H NMR (CDCI₃) δ 7.37-7.12 (m, 15H), 4.13 (m, 1H), 2.60 (m, 2H). The sulfide was deprotected using a solution of TFA in dichloromethane (1:1) for 2 hr. The solvents were removed under reduced pressure and the crude product was used in the next reaction.

**N-d**_{**3**}**-acetyl-S-(2-phenylpropan-3-ol)-L-cysteine**. The d₃-Nac-alcohol was formed using methodology described previously except that N-d₃-acetyl-cysteine was used in the formation of the d₃-Nac-atropaldehyde intermediate (2). The isotopic purity of the product was determined to be ≥ 95% as assessed by ¹H NMR and LC/MS. ¹H NMR (D₂0): δ 2.66-3.0 (m, 6H), 3.74 (t, 1H, J = 5.4 Hz), 4.4 (m, 1H), 7.3 (m, 5H). LC/MS: MH⁺ = 301.3.

**N-d**_{**3**}**-acetyl-S-(2-phenylpropanoic acid)-L-cysteine**. The d₃-Nac-acid was formed using methodology described previously except that N-d₃-acetyl-cysteine was used in the reaction with 2-phenyl acrylic acid (2). The isotopic purity of the product was determined to be ≥95% as assessed by ¹H NMR and LC/MS. ¹H NMR (D₂0): δ 2.75-3.23 (m, 4H), 3.82 (t,1H, J = 5.5 Hz), 4.4 (m, 1H), 7.32 (m, 5H). LC/ESI-MS: MH⁺ = 315.2.

**Preparation of Patient Urine Samples.** Urine samples were obtained from patient volunteers undergoing felbamate therapy for control of epileptic seizures and under the care of physicians at either the University of Virginia (Charlottesville, Virginia) or the EpiCare Center (Memphis, Tennessee). Urine samples were diluted four fold with distilled water (this was done prior to overnight shipment for samples requiring shipping) and placed in an orbital shaking water bath for ∼ 20 min at 37 °C to insure that all of the analytes were in solution. 500 µL of this diluted sample were removed and added to 100 µL of a mixture of the four deuterated internal standards. The concentration of standards in the mixture resulted in the addition of 563 nmol d₄-felbamate, 140 nmol d₂-acid carbamate, 54.0 nmol d₃-Nac-alcohol, and 27.5 nmol d₃-Nac-acid to each 500 µL diluted urine sample. After mixing, 200 µL were removed and added to 20 µL of 20% H0Ac. This acidified sample was then applied to a preconditioned Waters "Oasis" solid phase extraction cartridge (Waters Corp., Woburn, MA). The cartridge was washed with 2 mL 0.1% HOAc followed by 3 mL 10% CH₃CN/90% water (0.1 %) H0Ac. The analytes and internal standards were then eluted with 3 mL 30% CH₃CN:70% (0.1 %) HOAc. This fraction was then analyzed by LC/MS without further manipulation.

**LC/MS Analysis of Urine Samples for Metabolite Quantification.** LC/MS analysis was performed using a Waters 2690 HPLC equipped with an autosampler and a Waters 486 tunable absorbance detector. This LC system was interfaced to a Finnigan MAT LCQ ion trap mass spectrometer. A 10 µL injection of the fraction from solid phase extraction was applied to a Waters Symmetry C₈ reversed phase column (33% CH₃CN:67% (0.1%) H0Ac, 2.1 mm x 150 mm, 0.2 mL/min). The post-column flow was directed through a Waters 486 tunable absorbance detector (10 µL flow cell, λ = 214 nm) which was used for qualitative assessment of the sample and not for quantitation. The flow was then directed to the electrospray ionization source of the LCQ.

The mass spectrometer was programmed to collect data in full scan mode from 190 - 320 m/z and was tuned to maximize the signal from the analytes under the HPLC conditions. The values for the electrospray parameters were as follows: heated capillary temperature = 180°C; spray voltage = 5.6 kV; capillary voltage = 20 V; sheath gas (nitrogen) flow rate = 70; auxillary gas (helium) flow rate = 20. The automatic gain control (AGC) was on with a target ion count of 7 x 10⁷ ions and a maximum ion inject time of 150 ms. The scan time was ∼ 0.5 s. Linear response curves were observed for each analyte and internal standard pair over approximately two orders of magnitude range centered on the absolute amount of each internal standard added to the samples. The amount of analytes in the patient urine samples fell within these linear response ranges.

Quantification was achieved by integration of the peaks from the mass chromatogram for each analyte using software (Navigator 1.1) provided with the LCQ. The area (expressed as counts x seconds) of the peak for each metabolite was compared to the area for its corresponding deuterated internal standard. As the absolute amount of internal standard added per mL of urine was known, the absolute amount of analyte per mL of urine could be determined.

### Results

Analysis was performed on 34 urine samples from 31 patients undergoing felbamate therapy for control of epileptic seizures. As is common for epileptics, many of these patients (n = 19) were undergoing polytherapy for seizure control with the remainder (n = 12) undergoing felbamate monotherapy. The age of this patient pool (14 men and 17 women) spanned from 10-57 years old with a mean age of 37. All of the urine samples analyzed were found to contain both of the mercapturic acids, indicating that formation of atropaldehyde *in vivo* does occur in the patient population and appears to be universal. More Nac-alcohol 7 was excreted than Nac-acid 8 for all of the patients. The average ratio of Nac-alcohol to Nac-acid was 6.4, however the values varied widely (ratios = 2- 14).

The absolute amounts of the analytes excreted per mL of urine varied considerably between patients. For example, the amount of felbamate excreted ranged from 819 ± 8.5 nMoles/mL (this patient had a total daily dose of 3.6 grams of felbamate) to 10,064 ± 515 nMoles/mL (this patient had a total daily dose of 6.0 grams of felbamate). This illustrates the effect of urine volume on the results obtained from this method. Though the dosage difference was less than double, the difference in amount of felbamate excreted per mL was greater than ten times. Thus, to compare the results from one patient to another, the values for the metabolites were normalized to the amount of felbamate.

### Discussion

We have developed an isotope-dilution based LC/MS method to quantify the amounts of felbamate 1, the acid carbamate 4, and the two mercapturic acids 7 and 8 excreted in patient urine. Although the FDA has recommended patients be given felbamate therapy when other therapies have failed, it is estimated that 8,000- 12,000 patients remain on felbamate therapy in the United States (12).

Scheme I illustrates the metabolic pathway leading to atropaldehyde formation and its disposition. The aldehyde carbamate represents the "commitment" step. It is at this point that a molecule is either committed to the toxic pathway of atropaldehyde 5 or the detoxification pathway of the acid carbamate 4. The amount of mercapturic acids 7 and 8 excreted in urine will mirror the flux through the "toxic" pathway. That is, an individual that generates high levels of atropaldehyde would be expected to have correspondingly high levels of the mercapturic acids. Therefore, the ratio of acid carbamate excreted compared to the combined mercapturic acids would describe the disposition of the aldehyde carbamate for a given patient. The values for the two mercapturic acids can be combined as they both represent the same pathway of aldehyde carbamate disposition. Of course, there are other factors that may modulate the disposition through these two pathways (i.e., co-administration of modulators of enzyme activity or glutathione levels ), but this appears to be a promising approach to evaluating the metabolic distribution between toxic and non-toxic pathways in a patient population.

We applied this LC/MS method to the analysis of 34 urine samples from 31 patients undergoing felbamate therapy for control of epileptic seizures. All of these patients have been undergoing felbamate therapy for several years, without any of the severe side effects. As the severe toxicities associated with felbamate demonstrate a mean onset time of ≤6 months, these patients probably constitute a population of "normal" or "safe"metabolizers of felbamate.

The data obtained from the urine samples is illustrated that there is a "normal" range for the distribution of the aldehyde carbamate between the acid carbamate and atropaldehyde (as represented by the mercapturic acids). There does not appear to be a significant correlation between sex or therapy type and the relative amounts of metabolites formed. An individual with high esterase activity would produce relatively more of the monocarbamate alcohol from felbamate leading to increased generation of all subsequent metabolites. However, the ratio of the metabolites may still be very similar.

By our hypothesis, alkylation of proteins by atropaldehyde may result in the generation of antigens that precipitate an immune response in a manner similar to mechanisms of immune mediated toxicity for other agents. If the toxicity due to atropaldehyde is immune mediated, then susceptibility to this toxicity will be a function not only of the formation of atropaldehyde-protein conjugates but also of a patient's immune system phenotype. Some patients may have a particular phenotype that makes them allergic to atropaldehyde-protein conjugates while others do not exhibit this response. Alternatively, everyone may have the potential for an immune response, but the amount of atropaldehyde protein conjugates produced must reach a critical level before the immune response occurs. That is, all of the patients may be producing low levels of antigens, but the levels are not normally high enough to trigger an immune response. It is not until a secondary event, such as inhibition of acid carbamate formation or glutathione depletion, occurs that the production of antigens surpasses the critical level and toxicity is manifest.

Because of the potential for an immune mediated component for felbamate toxicity by our hypothesis, it may be important to understand both an individual's level of atropaldehyde formation and their phenotype to develop a complete screening method. The method described above has demonstrated sufficient precision for the identification of "outliers" and appears to hold potential for the monitoring of patients undergoing felbamate therapy.

### Example 2

### Synthesis of Febamate Derivatives

### Materials and Methods

**Chemicals and Instruments.** All reagents were purchased from Aldrich Chemical Co. and were of the highest quality available. HPLC was performed on a Waters 2690 Separations Module with a Waters 484 tunable absorbance detector (at 214 nm) using a Waters Symmetry C₁₈ (2.1 mm x 150 mm) column. Mass Spectra were obtained by coupling this LC system to a Finnigan MAT LCQ ion trap mass spectrometer equipped with an electrospray ionization source. NMR spectra were recorded on a General Electric QE300 spectrometer at 300 MHz and chemical shifts are reported in ppm. Melting points were determined on a Thomas-Hoover UNI-MELT apparatus and are uncorrected.

### Synthesis

**2-Fluoro-2-phenyl-1,3-propanediol.** 2-Fluoro-2-phenyl-1, 3-propanediol was obtained from diethyl 2-fluoro-2-phenylmalonate by lithium aluminum hydride reduction using methodology analogous to that described previously for the formation of 2-phenyl-1, 3-propanediol. (Thompson etal., *Chem*. *Res. Toxicol.* **9**, 1225-1229 (1996)). However, the reduction was initiated at - 40 °C and was allowed to warm to room temperature over one hour, followed by stirring for an additional 1 to 2 hours, at which time the reaction was complete by TLC. ¹H NMR (CDCl₃): δ 7.43-7.3 (m, 5H), 4.01 (dq, 4H, *J* = 22.7, 12.3 Hz), 2.82 (bs, 2H), ¹³C NMR (CDCl₃): 138.2, 129.1, 129.1, 128.9, 125.3, 125.2, 100.4, 98.0, 67.0, 66.7.

**2-Fluoro-2-phenyl-1, 3-propanediol monocarbamate (13, x = Fluoro).** The title compound was prepared from the 2-fluoro-2-phenyl-1, 3-propanediol using methodology described previously. (Thompson etal., *Chem*. *Res. Toxicol.* **9**,1225-1229 (1996)). The product thus obtained was recrystalized from ethyl ether and the purity of the product was determined to be 98% as assessed by ¹H NMR and LC/MS (72 % yield, R_{*f*}_{(ethyl ether)} = 0.25, mp = 67 - 69 °C). ¹H NMR ((CDCl₃): δ 7.4 - 7.2 (m, 5H), 5.05 (bs, 2H), 4.52 (ddd, 2H, *J* = 20.2, 12.5, 6.5 Hz), 3.9 (dd, 2H, *J* = 12.5,6.5 Hz). LC/ESI-MS: MH⁺ = 214.

**3-Carbamoyl-2-fluoro-2-phenylpropionic acid (17, x = Fluoro).** 3-Carbamoyl-2-fluoro-2-phenylpropionic acid 17 was prepared from 2-fluoro-2-phenyl-1, 3-propanediol monocarbamate 13 following the procedure of Adusumalli et al, which describes the preparation of 3-carbamoyl-2-phenylpropionic acid (85 % yield, R_{*f*}_{(ethyl ether)} = 0.05). ¹H NMR ((CDCl₃): δ 9.1 (bs, 1H), 7.6-7.2 (m, 5H), 5.4 (bs, 2H), 4.8 (dd, 1H, *J* = 10, 7 Hz), 4.72 (t, 1H, *J* = 7 Hz).

**2-Fluoro-2-phenyl-1, 3-propanediol dicarbamate (12, x = Fluoro).** The title compound, fluoro felbamate 12, was made from 2-fluoro-2-phenyl-1, 3-propanediol by the method of Adusumalli *et al*, which describes the preparation of felbamate *Drug*. *Metab. Disp.* **21**, 710-716 (1993). (82 % yield, R_{*f*}_{(ethyl ether)} = 0.20, mp = 69 - 72 °C). ¹H NMR ((CDCl₃): δ 7.4-7.3 (m, 5H), 5.2 (bs, 4H), 4.48 (dq, 4H, *J* = 20.8, 14.2 Hz). C, 56.33; H, 5.67; N, 6.57 found C, 56.24; H, 5.55; N, 6.52.

**5-Fluoro-5-phenyl-1, 3-oxazinane-2,4-dione (16, x = Fluoro).** 3-carbamoyl-2-fluoro-2-phenyl propionic acid (17, 0.1 mmol) and 1,1'-carbonyldiimidizole (0.25 mmol) were dissolved in dichloromethane (5 mL) and the resultant solution was magnetically stirred for 12 hours in a sealed vessel.¹² The mixture was purified by passage of the crude reaction mixture through a pad of silica gel (10 g) in a fritted funnel, eluting with ethyl ether, affording, after recrystalization from ethyl ether, the title compound as a white powder in 42 % yield (R_{*f*}_{(ethyl ether)} = 0.70, mp = 115 - 117 °C). ¹H NMR (CDCl₃): δ 7.48 - 7.43 (m, 3H), 7.42 - 7.37 (m, 2H), 4.93 (dd, 2H, *J* = 24.6,12.7 Hz), 4.68 (t, 2H, *J =* 13 Hz) LC/ESI-MS: MH⁺ = 210. C, 57.42; H, 3.85; N, 6.70 found C, 57.23; H, 3.88; N, 6.67.

### Example 3

### Neuroprotection and toxicity assays

The fluoro derivatives synthesized were submitted to the National Institute of Neurological Disorders and Stroke (NINDS) for evaluation in a standard panel of assays. Two convulsant tests (MES and scMET), and a toxicity screen (rotorod in mice, positional sense and gait in rats) were employed for evaluating the compounds activity as neuroprotecting agents. These testing procedures are described in detail in *Molecular and Cellular Targets for Anti-epileptic Drugs*, eds. G. Avanzini, G. Regesta, P. Tanganelli, M. Avoli, 1997, John Libbey & Company Ltd, pp 191-198. The felbamate derivatives were evaluated for anticonvulsant activity following intraperitoneal (i.p.) administration in mice and oral administration in rats.

### The Maximal Electroshock Seizure (MES)

The MES test is a model for generalized tonic-clonic seizures and is used to identify compounds which prevent seizure spread. The behavioral and electrographic seizures generated in this model are consistent with the human disorder. In the MES test, an electrical stimulus of 0.2 s in duration (50 mA in mice and 150 mA in rat at 60Hz) was delivered via corneal electrodes primed with an electrolyte solution containing an anesthetic agent. Mice were tested at 30 minutes and 4 hours following doses of 30, 100 and 300 mg/kg of test compound. Rats were tested at time intervals between 0.25 and 4 hours following a standard oral dose of 30 mg/kg. Abolition of the hindlimb tonic extensor component indicates the test compound's ability to inhibit MES-induced seizure spread.

### The Subcutaneous (Metrazol) Seizure Test (scMET)

This is a model that primarily identifies compounds that raise seizure threshold. With some minor exceptions, the pharmacological profile of the scMET seizure model is consistent with the human condition. The scMET test utilizes a dose of pentylenetetrazol (85 mg/kg in Carworth Farms No. 1 mice and 70 mg/kg in Sprague-Dawley rats) that induces clonic seizures lasting for a period of at least five seconds in 97 % (CD97) of animals tested. At the anticipated time of testing the convulsant is administered subcutaneously. The test compound is administered intraperitoneally in mice and orally in rats. Animals are observed over a 30 minute period. Absence of clonic spasms in the observed time period indicates a compound's ability to abolish the effect of pentylenetetrazol on seizure threshold. All clinically active anticonvulsants have been found to be protective in at least one of these two tests.

### Minimal neurotoxicity

Toxicity induced by a compound is detected in mice using the standardized rotorod test described by Dunham & Miya (1957). Untreated control mice, when placed on a 6 r.p.m. rotation rod, can maintain their equilibrium for a prolonged period of time. Neurological impairment can be demonstrated by- the inability of a mouse to maintain equilibrium for one minute in each of three successive trials.

Rats are examined for behavioral toxicity by the positional sense test and a gait and stance test. In the positional sense test, one hind leg is gently lowered over the edge of a table, whereupon the rat, experiencing neurological deficit, will fail to lift its leg quickly back to a normal position. In the gait and stance test, neurotoxicity is indicated by a circular or zigzag gait, ataxia, abnormal spread of the legs, abnormal posture, tremor hyperactivity, lack of exploratory behavior, somnolence, stupor or catalepsy.

### Results

The results of these evaluations (MES, scMET and toxicity (TOX)) are summarized in Tables 1-6. For the MES and scMET tests the data is presented in the form of the number of animals protected by the administered compound / total number of animals tested. For the toxicity test, data is presented in the form of the number of animals exhibiting toxic effects / total number of animals tested. Qualitative data was obtained for fluoro felbamate **12** (Tables 1 and 2A), fluoro monocarbamate felbamate **13** (Tables 1 and 2B), and fluorodioxo **16** (Tables 1 and 2C) and for cyclic felbamate **21** (Tables 5 and 6B) and cyclic monocarbamate 22 (Tables 5 and 6A). More extensive quantitative data was also obtained for fluoro felbamate **12** in mice (Table 3) and rats (Table 4).

Many of the agents proposed herein are derived from felbamate 1 or it's metabolites and should possess greater metabolic stability than the corresponding parent agents. Fluoro felbamate **12** and fluoro monocarbamate felbamate **13** each show anti-seizure activity and neither appears to exhibit high levels of toxicity. Surprisingly, fluoro felbamate **12** was found to be approximately 5-10 times more active than felbamate.

Fluoro felbamate **12** showed MES protective effects at 30 mg/kg at both 30 minutes and at 4 hours (see Table 1). There are some toxic effects observed at that dosage at the 30 minute timepoint but not at 4 hours. Toxicity across time doe not occur until dosages of 300mg/kg. As shown in Table 2A, fluoro felbamate 12 provided full protectionat 30 mg/kg over several timepoints. Quantitative results obtained for fluoro felbamate 12 in mice indicated a MES ED50 of approximately 20 mg/kg with a safety ratio of at least five (see Table 3). Results obtained for oral administration of fluoro felbamate **12** to rats indicated a MES ED50 of about 3 mg/kg with no toxicity at doses up to 500 mg/kg. Finally, oral administration of fluoro felbamate **12** to mice indicated a MES ED50 of about 27 mg/kg with no toxicity at doses up to 218 mg/kg.

Fluoro monocarbamate felbamate 13 showed moderate MES protection at a dosage of 100 mg/kg in mice (see Table 1). Orally protection is seen at several different timepoints at a dosage level of 30 mg/kg (see Table 2B).

Interestingly, fluoro oxazinane-dione **16** was found to be inactive in mice but possesses strong anti-seizure activity in rats (Tables 1 and 2C). The preliminary evaluation of Cyclic-MCF **22** and Cyclic-Felbamate **21** is summarized in Tables 5, 6A and 6B. These agents also displayed potent activity in rat models.

**Table 1.**

| **Qualitative Evaluation of Fluoro Derivatives in Mouse.** | | | | | | | |
|---|---|---|---|---|---|---|---|
| Mice I. P. | | fluoro felbamate | | fluoro monocarbamate felbamate | | fluoro dioxo | |
| Test | Dose | Time | | Time | | Time | |
| | mg/kg | 0.5h | 4.0h | 0.5h | 4.0h | 0.5h | 4.0h |
| MES | | | | | | | |
| | 3 | 0/4 | 0/1 | | | | |
| | 10 | 0/4 | 1/4 | | | | |
| | 30 | 1/1 | 1/1 | 0/1 | 0/1 | 0/1 | 0/1 |
| | 100 | 3/3 | 3/3 | 2/3 | 0/3 | 0/3 | 0/3 |
| | 300 | 1/1 | 1/1 | 1/1 | 0/1 | 0/1 | 0/1 |

| ScMET | | | | | | | |
|---|---|---|---|---|---|---|---|
| | 30 | 0/1 | 0/1 | 0/1 | 0/1 | 0/1 | 0/1 |
| | 100 | 0/1 | 0/1 | 0/1 | 0/1 | 0/1 | 0/1 |
| | 300 | 0/1 | 0/1 | 1/1 | 0/1 | 0/1 | 0/1 |

| TOX | | | | | | | |
|---|---|---|---|---|---|---|---|
| | 3 | 0/4 | 0/4 | | | | |
| | 10 | 0/4 | 0/4 | | | | |
| | 30 | 1/4 | 0/2 | 0/4 | 0/2 | 0/4 | 0/2 |
| | 100 | 4/8 | 0/4 | 3/8 | 0/4 | 0/8 | 0/4 |
| | 300 | 4/4 | 2/2 | 2/4 | 0/2 | 1/4 | 0/2 |

**Table 2A.**

| **Qualitative Evaluation of Fluoro Derivatives in Rat.** | | | | | | |
|---|---|---|---|---|---|---|
| Rat (p.o.) | | fluoro felbamate | | | | |
| Dose | | Time | | | | |
| Test | mg/kg | 0.25h | 0.5h | 1.0h | 2.0h | 4.0h |
| MES | 30 | 2/4 | 4/4 | 4/4 | 4/4 | 4/4 |
| TOX | 30 | 0/4 | 0/4 | 0/4 | 0/4 | 1/4 |

**Table 2B.**

| **Qualitative Evaluation of Fluoro Derivatives in Rat.** | | | | | | |
|---|---|---|---|---|---|---|
| Rat (p.o.) | | fluoro monocarbamate felbamate | | | | |
| Dose | | Time | | | | |
| Test | mg/kg | 0.25h | 0.5h | 1.0h | 2.0h | 4.0h |
| MES | 30 | 0/4 | 3/4 | 0/4 | 1/4 | 0/4 |
| TOX | 30 | 0/4 | 0/4 | 0/4 | 0/4 | 0/4 |

**Table 2C.**

| **Qualitative Evaluation of Fluoro Derivatives in Rat.** | | | | | | |
|---|---|---|---|---|---|---|
| Rat (p.o.) | | fluoro dioxo | | | | |
| Dose | | Time | | | | |
| Test | mg/kg | 0.25h | 0.5h | 1.0h | 2.0h | 4.0h |
| MES | 30 | 0/4 | 1/4 | 1/4 | 1/4 | 2/4 |
| TOX | 30 | 0/4 | 0/4 | 0/4 | 0/4 | 0/4 |

**Table 3.**

| **Quantitative Evaluation of Fluoro Felbamate in Mouse.** | |
|---|---|
| Test (I.P.) | ED50 (mg/kg) |
| MES | 19.96 |
| ScMET | > 160.00 |
| TOX | 115 |

**Table 4.**

| **Quantitative Evaluation of Fluoro Felbamate in Rat.** | |
|---|---|
| Test (p.o.) | ED50 (mg/kg) |
| MES | 3.0 |
| ScMET | >250.00 |
| TOX | >500 |

**Table 5.**

| **Qualitative Evaluation of Fluoro Derivatives in Mouse.** | | | | | |
|---|---|---|---|---|---|
| Mice I. P. | | Cyclic MCF | | cyclic felbamate | |
| Dose | | Time | | Time | |
| Test | mg/kg | 0.5h | 2.0h | 0.5h | 4.0h |
| MES | 30 | 0/1 | 0/1 | 0/1 | 0/1 |
| | 100 | 0/3 | 2/3 | 0/3 | 0/3 |
| | 300 | 1/1 | 1/1 | 0/1 | 0/1 |
| ScMET | 30 | 0/1 | 0/1 | 0/1 | 0/1 |
| | 100 | 2/5 | 0/1 | 0/1 | 0/1 |
| | 300 | 2/5 | 0/1 | 0/1 | 0/1 |
| TOX | 30 | 0/4 | 0/2 | 0/4 | 0/2 |
| | 100 | 0/8 | 0/4 | 0/8 | 0/4 |
| | 300 | 0/4 | 0/2 | 0/4 | 0/2 |

**Table 6A.**

| **Qualitative Evaluation of Fluoro Derivatives in Rat.** | | | | | | |
|---|---|---|---|---|---|---|
| Rat (p.o.) | | Cyclic MCF | | | | |
| Dose | | Time | | | | |
| Test | mg/kg | 0.25h | 0.5h | 1.0h | 2.0h | 4.0h |
| MES | 30 | 0/4 | 0/4 | 1/4 | 2/4 | 3/4 |
| TOX | 30 | 0/4 | 0/4 | 0/4 | 0/4 | 0/4 |

**Table 6B.**

| **Qualitative Evaluation of Fluoro Derivatives in Rat.** | | | | | | |
|---|---|---|---|---|---|---|
| Rat (p.o.) | | cyclic felbamate | | | | |
| Dose | | Time | | | | |
| Test | mg/kg | 0.25h | 0.5h | 1.0h | 2.0h | 4.0h |
| MES | 30 | 2/4 | 2/4 | 3/4 | 0/4 | 3/4 |
| TOX | 30 | 0/4 | 0/4 | 0/4 | 0/4 | 0/4 |

### Discussion

The panel of proposed agents (Scheme II), several of which are structural variants of felbamate **1** and it's metabolites, seeks to address the occurrence of idiosyncratic adverse reactions that have been associated with the use of felbamate **1**. The results obtained from the evaluation of five of these agents have demonstrated that these agents possess anti seizure activity and they have not, as of yet, exhibited toxicity at therapeutically relevant doses. As is normal for agents that target neurological disorders, these agents have elicited minor levels of neurotoxicity at high doses, but there appears to exist a reasonable therapeutic index. Additionally, the activity of these agents, specifically fluoro felbamate 12 and fluoro monocarbamate felbamate 13, correlates well with the activity of the unfluorinated parent agents, felbamate 1 and monocarbamate felbamate 2, respectively. The evaluation of Cyclic-MCF 22 and Cyclic-Felbamate 21 also demonstrated these agents to be effective in the attenuation of seizures.

### Conclusion

Given the success of the evaluation of fluoro felbamate 12, fluoro monocarbamate felbamate 13, fluoro oxazinane-dione 16, Cyclic-MCF 22 and Cyclic-Felbamate 21 this panel of agents represents a new class of neuro-active entities that is designed to preclude the formation of putative toxic species that may be associated with the occurrence of adverse reactions observed with the use of felbamate. Taken as a whole, this proposed panel of agents addresses the presumed relevant metabolic processes, namely metabolism to atropaldehyde and metabolism by certain members of the cytochrome P450 metabolic family.

### Example 4

### The Hippocampal Kindling Model

The hippocampal kindling model can be used to evaluate a compound's ability to affect both the expression and acquisition of focal seizures. The hippocampal kindling paradigm as described by Lothman and Williamson (Lothman, 1994) allows the temporal effects of a drug to be evaluated in a single animal. This procedure requires the surgical placement ofbipolar electrodes in the ventral hippocampus of adult male Sprague-Dawley rats. Stage five behavioral seizures are produced by using a stimulus consisting of a 50 Hz, 10 s train of 1 ms biphasic 200 uA pulses delivered every 30 min for 6 hours (12 stimuli per day) on alternating days for a total of 60 stimulations (five stimulus days). Prior to evaluating a candidate's anticonvulsant activity, a drug free control period consisting of supramaximal stimulations are recorded to verify the stability of a stage five generalized seizure.

A single dose of the candidate compound is then administered intraperitoneally (i.p.), 15 min following the last control stimulation. The anticonvulsant activity of the drug is assessed every 30 min for three to four hours starting 15 min after administering the test material. After each stimulation, individual Racine seizure scores and afterdischarge durations are recorded. Rats are used again in drug trials after four to five drug- and stimulus-free days.

In the kindling acquisition study, drugs are tested for their ability to prevent the development of the kindled state in electrode implanted rats. The candidate compound is administered during the kindling procedure and at a predetermined time prior to the electrical stimulus. The dosing interval and the dose of the drug are based on the compound's activity observed in the acute seizure expression studies. Results from drug-treated animals are compared to those of saline-treated rats.

This treatment is repeated on stimulus days two, three, four, and five. After a stimulus-free interval of one week, the effect of prior drug treatment on kindling acquisition is assessed by challenging the animal with the kindling stimulus protocol. The standardized kindling protocol is then carried out with the behavioral seizure score and afterdischarge duration recorded for each rat during three 'retest days'. Saline treated rats are fully kindled at the first stimulation following the one week stimulus-free period. An active compound would be expected to lower behavioral scores and afterdischarge duration compared to saline control rats. The suppression or lengthening of the delay in the acquisition of the kindled response may indicate that the candidate compound can act to prevent the development of seizures. Such compounds could be termed 'antiepileptogenetic'.

### Results

The kindled results for fluoro felbamate **12** are presented in Table 7. At a dose of 100mg/kg a significant drop in seizure score was observed along with a corresponding decrease in afterdischarge duration.

**Table 7.**

| **Hippocampal Kindled Rats** Solvent: MC (M&P, SB) Route: i.p. | | |
|---|---|---|
| Dose: 25 mg/kg | fluoro felbamate | . |
| Time (min) | Seizure Score ± S.E.M. | Afterdischarge Duration (sec) ± S.E.M |
| Control | 4.71 ± 0.18 | 73.14 ± 4.61 |
| 15 | 4.86 ± 0.14 | 77.29 ± 4.91 |
| 45 | 4.57 ± 0.20 | 71.43 ± 5.50 |
| 75 | 4.00 ± 0.69 | 64.43 ± 11.19 |
| 105 | 4.57 ± 0.20 | 70.29 ± 4.85 |
| 135 | 4.43 ± 0.57 | 53.86 ± 9.33 |
| 165 | 4.71 ± 0.18 | 59.29 ± 3.28* |
| 195 | 3.86 ± 0.67 | 54.00 ± 9.62 |
| 225 | 4.00 ± 0.69 | 53.29 ± 9.03 |
| 255 | 4.86 ± 0.14 | 64.00 ± 3.42 |

| Dose: 50 mg/kg | | |
|---|---|---|
| Control | 5.00 ± 0.00 | 83.71 ± 6.97 |
| 15 | 3.86 ± 0.70 | 67.14 ± 12.39 |
| 45 | 2.86 ± 0.83* | 68.57 ± 20.14 |
| 75 | 3.14 ± 0.83* | 63.00 ± 17.45 |
| 105 | 3.43 ± 0.90 | 60.86 ± 14.93 |
| 135 | 3.71 ± 0.64* | 65.00 ± 13.10 |
| 165 | 2.71 ± 0.97* | 43.14 ± 14.67* |
| 195 | 3.14 ± 0.83 | 52.86 ± 14.59 |
| 225 | 3.00 ± 0.87* | 39.86 ± 10.98* |
| 255 | 2.57 ± 0.72* | 33.29 ± 11.76* |

**Table 7.**

| **Hippocampal Kindled Rats (continued)** Solvent: MC (M&P, SB) Route: i.p. | | |
|---|---|---|
| Dose: 100 mg/kg | | |
| Control | 5.00 ± 0.00 | 76.00 ± 5.03 |
| 15 | 1.75 ± 0.73* | 40.75 ± 15.74 |
| 45 | 1.50 ± 0.57* | 29.00 ± 10.86* |
| 75 | 1.88 ± 0.67* | 38.63 ± 12.58* |
| 105 | 2.38 ± 0.65* | 53.38 ± 12.59 |
| 135 | 2.00 ± 0.76* | 33.63 ± 12.88* |
| 165 | 2.75 ± 0.65* | 43.38 ± 12.79* |
| 195 | 2.25 ± 0.70* | 45.25 ± 13.33* |
| 225 | 2.50 ± 0.73* | 47.38 ± 14.80 |
| 255 | 2.50 ± 0.65* | 45.38 ± 13.92 |

| | | |
|---|---|---|
| * Significantly different from control. | | |

| **Dose Responses** | | | |
|---|---|---|---|
| Dose (mg/kg) | Number Protected / Number Tested | Seizure Score ± S.E.M. | Afterdischarge Duration (sec) ± S.E.M. |
| 25 | 0/7 | 4.57 ± 0.20 | 71.43 ± 5.50 |
| 50 | 3/7 | 2.86 ± 0.83* | 68.57 ± 20.14 |
| 100 | 7/8^{a} | 1.50 ± 0.57* | 29.00 ± 10.86* |

| | | | |
|---|---|---|---|
| ^{a} 2/8 toxic | | | |
| * Significantly different from control. | | | |

All publications, patents, and patent documents are incorporated by reference herein, as though individually incorporated by reference. The invention has been described with reference to various specific and preferred embodiments and techniques. However, it should be understood that many variations and modifications may be made while remaining within the spirit and scope of the invention.

## Claims

1. Use of a compound of formula (I), (II), or (III): 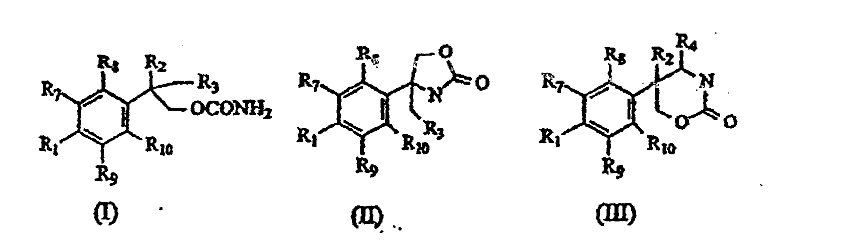 wherein:
R₁, R₇, R₈, R₉ and R₁₀ are each independently H, halo, alkyl, haloalkyl, NR₅R₆, hydroxy, or alkoxy;
R₂ is halo;
R₃ is hydroxy or -OCONH₂;
R₄ is hydroxy or carbonyl; and
R₅ and R₆ are each independently alkyl
or a pharmaceutically acceptable salt thereof for the preparation of medicament for the treatment of neuropathic pain.

2. The use of claim 1 wherein R₁, R₇ and R₈ are each independently H, halo, alkyl, haloalkyl or hydroxy; R₂ is fluoro; R₃ is hydroxy or -OCONH₂; R₄ is hydroxy or carbonyl; and R₉ and R₁₀ are each H.

3. The use of claim 2 wherein a compound of formula (II) or (III); or a salt thereof is administered.

4. The use of claim 3 wherein R₇ and R₈ are each H; R₁ is H or F; and R₄ is hydroxy or carbonyl.

5. The use of claim 1 or 2 wherein a compound of formula (I) or a salt thereof is administered; wherein R₁ is halo, haloalkyl, or hydroxy; R₇ and R₈ are each independently H, halo, alkyl, haloalkyl, or hydroxy; and R₃ is hydroxy or -OCONH₂.

6. The use of claim 5 wherein R₇ and R₈ are each H; R₁ is F; and R₃ is hydroxy or -OCONH₂.

7. The use of claim 1 or 2 wherein a compound of formula (I) or a salt thereof is administered; wherein R₁ is H, halo, haloalkyl, or hydroxy; R₇ and R₈ are each independently H; aud R₃ is hydroxy or -OCONH₂.

8. The use of claim 7 wherein R₁ is H; and R₃ is -OCONH₂.

9. The use of claim 1 or 2 wherein a compound of formula (III) or a pharmaceutically acceptable salt thereof is administered; wherein R₁ and R₇ are each independently H, halo, haloalkyl and hydroxy; R₈ is H; and R₄ is hydroxy or carbonyl.

10. The use of claim 9 wherein R₇ is H.

11. The use of claim 7 wherein R₁ is H or F.

12. The use of claim 10 wherein R₁ is H or F.

13. Use of a compound of formula (I), (II), or (III), or a pharmaceutically acceptable salt thereof, as defined in any one of claims 1-12. for the preparation of a medicament for the treatment of obesity

14. The use of any one of claims 1, 2 or 13 wherein the medicament is in unit dosage form that comprises from about 0.1 mg/kg to about 1 g/kg of the compound

15. Use of a compound of formula (I), (II), or (III), or a pharmaceutically acceptable salt thereof, as defined in any one of claims 1-12 for the preparation of a medicament for the treatment of glaucoma.

16. The use of claim 15 wherein the medicament is in unit dosage form comprising about 1.0 mg/kg to about 1 g/kg of said compound

17. Use of a compound of formula (I), (II), or (III), or a pharmaceutically acceptable salt thereof, as defined in any one of claims 1-12 for the preparation of a medicament for the treatment of depression.

18. Use of a compound of formula (I), (II), or (III), or a pharmaceutically acceptable salt thereof, as defined in any one of claim 1-12 for the preparation of a medicament for the treatment of mood disorders.

19. Use of a compound of formula (I), (II), or (III), or a pharmaceutically acceptable salt thereof, as defined in any one of claim 1-12 for the preparation of a medicament for the treatment of diabetic neuropathy

20. Use of a compound of fomaula (I), (II), or (III), or a pharmaceutically acceptable salt thereof, as defined in any one of claim 1-12 for the preparation of a medicament for the treatment of Alzheimer's disease, Parkinson's disease or HIV dementia.

21. Use of a compound of formula (I), (II), or (III), or a pharmaceutically acceptable salt thereof, as defined in any one of claims 1-12 for the preparation of a medicament for the treatment of Meningitis, CNS Vasculitis, Adrenoleukodystrophy, Impotence, Schizophrenia, Drug Addiction, Multiple Sclerasis, Fatigue, Lead Poisoning. Mitochondrial Myopathies, HIV Dementia, Amyotrophic Lateral Sclerosis, Attention Deficit Disorder, Narcolepsy, Childbirth Complications, Surgical Anesthesia, Traumatic Head and Spinal Cord Injury, Hypoglycemia, Tourette's Syndrome or Hepatic Encephalopathy

## Patentansprüche

1. Verwendung einer Verbindung der Formel (I), (II) oder (III): 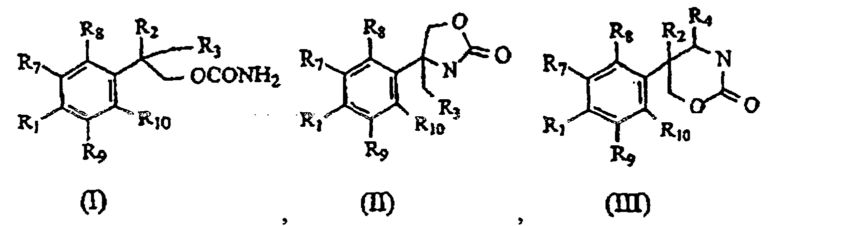 worin:
R₁, R₇, R₈, R₉ und R₁₀ jeweils unabhängig H, Halogen, Alkyl, Halogenalkyl, NR₅R₆, Hydroxy oder Alkoxy sind;
R₂ Halogen ist;
R₃ Hydroxy oder -OCONH₂ ist;
R₄ Hydroxy oder Carbonyl ist; und
R₅ und R₆ jeweils unabhängig Alkyl sind
oder eines pharmazeutisch verträglichen Salzes hiervon zur Herstellung eines Medikaments zur Behandlung von neuropatischem Schmerz.

2. Verwendung nach Anspruch 1, wobei R₁, R₇ und R₈ jeweils unabhängig H, Halogen, Alkyl, Halogenalkyl oder Hydroxy sind; R₂ Fluor ist; R₃ Hydroxy oder -OCONH₂ ist; R₄ Hydroxy oder Carbonyl ist; und R₉ und R₁₀ jeweils H sind.

3. Verwendung nach Anspruch 2, wobei eine Verbindung der Formel (II) oder (III) oder ein Salz hiervon verabreicht wird.

4. Verwendung nach Anspruch 3, wobei R₇ und R₈ jeweils H sind; R₁ H oder F ist und R₄ Hydroxy oder Carbonyl ist.

5. Verwendung nach Anspruch 1 oder 2, wobei eine Verbindung der Formel (I) oder ein Salz hiervon verabreicht wird, wobei R₁ Halogen, Halogenalkyl oder Hydroxy ist, R₇ und R₈ jeweils unabhängig H, Halogen, Alkyl, Halogenalkyl oder Hydroxy sind und R₃ Hydroxy oder -OCONH₂ ist.

6. Verwendung nach Anspruch 5, wobei R₇ und R₈ jeweils H sind; R₁ F ist und R₃ Hydroxy oder -OCONH₂ ist.

7. Verwendung nach Anspruch 1 oder 2, wobei eine Verbindung der Formel (I) oder ein Salz hiervon verabreicht wird, wobei R₁ H, Halogen, Halogenalkyl oder Hydroxy ist, R₇ und R₈ jeweils unabhängig H sind und R₃ Hydroxy oder -OCONH₂ ist.

8. Verwendung nach Anspruch 7, wobei R₁ H ist und R₃ -OCONH₂ ist.

9. Verwendung nach Anspruch 1 oder 2, wobei eine Verbindung der Formel (III) oder ein pharmazeutisch verträgliches Salz hiervon verabreicht wird, wobei R₁ und R₇ jeweils unabhängig H, Halogen, Halogenalkyl und Hydroxy sind; R₈ H ist und R₄ Hydroxy oder Carbonyl ist.

10. Verwendung nach Anspruch 9, wobei R₇ H ist.

11. Verwendung nach Anspruch 7, wobei R₁ H oder F ist.

12. Verwendung nach Anspruch 10, wobei R₁ H oder F ist.

13. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung von Fettleibigkeit.

14. Verwendung nach einem der Ansprüche 1, 2 oder 13, wobei das Medikament eine Einheitsdosierform ist, die etwa 0,1 mg/kg bis etwa 1 g/kg der Verbindung umfaßt.

15. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung von Glaukom.

16. Verwendung nach Anspruch 15, wobei das Medikament eine Einheitsdosierform ist, die etwa 1,0 mg/kg bis etwa 1 g/kg der Verbindung umfaßt.

17. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung von Depression.

18. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung von Stimmungsschwankungen.

19. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung von diabetischer Neuropathie.

20. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung der Alzheimerkrankheit, der Parkinsonkrankheit oder von HIV-Demenz.

21. Verwendung einer Verbindung der Formel (I), (II) oder (III) oder eines pharmazeutisch verträglichen Salzes hiervon, wie in einem der Ansprüche 1 bis 12 definiert, für die Herstellung eines Medikaments zur Behandlung von Meningitis, ZNS-Vaskulitis, Adrenoleukodystrophie, Impotenz, Schizophränie, Drogenabhängigkeit, Multiple Sklerose, Ermüdung, Bleivergiftung, mitochondriale Myopathien (Luft'sche Krankheit), HIV-Demenz, amyotrophe Lateralsklerose, Aufmerksamkeitsschwäche, Narkolepsie, Komplikationen bei der Geburt, chirurgischer Anästhesie, traumatische Kopf- und Rückenmarksverletzung, Hypoglykämie, Tourette-Syndrom oder Leberenzephalopathie.

## Revendications

1. Utilisation d'un composé de formule (I), (II) ou (III) ; 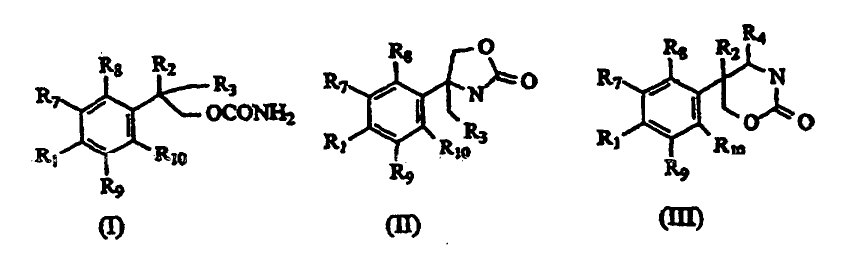 dans lesquelles :
R₁, R₇, R₈, R₉ et R₁₀ représentent chacun, indépendamment les uns des autres, un atome d'hydrogène, un atome d'halogène, un groupe alkyle, halogénoalkyle, NR₅R₆, hydroxyle ou alcoxy ;
R₂ représente un atome d'halogène ;
R₃ représente un groupe hydroxyle ou -OCONH₂ ;
R₄ représente un groupe hydroxyle ou carbonyle ; et
R₅ et R₆ représentent chacun, indépendamment l'un de l'autre, un groupe alkyle, ou d'un de ses sels acceptables sur le plan pharmaceutique pour la préparation de médicament pour le traitement d'une douleur neuropathique.

2. Utilisation selon la revendication 1, dans laquelle R₁, R₇ et R₈ représentent chacun, indépendamment les uns des autres, un atome d'hydrogène, un atome d'halogène, un groupe alkyle, halogénoalkyle ou hydroxyle ; R₂ représente un atome de fluor ; R₃ représente un groupe hydroxyle ou -OCONH₂ ; R₄ représente un groupe hydroxyle ou carbonyle ; et R₉ et R₁₀ représentent chacun un atome d'hydrogène.

3. Utilisation selon la revendication 2, dans laquelle un composé de formule (II) ou (III) ; ou un de ses sels est administré.

4. Utilisation selon la revendication 3, dans laquelle R₇ et R₈ représentent chacun un atome d'hydrogène ; R₁ représente un atome d'hydrogène ou de fluor ; et R₄ représente un groupe hydroxyle ou carbonyle.

5. Utilisation selon la revendication 1 ou 2 dans laquelle un composé de formule (I) ou un de ses sels est administré ; dans laquelle R₁ représente un atome d'halogène, un groupe halogénoalkyle ou hydroxyle ; R₇ et R₈ représentent chacun, indépendamment l'un de l'autre, un atome d'hydrogène, un atome d'halogène, un groupe alkyle, halogénoalkyle ou hydroxyle ; et R₃ représente un groupe hydroxyle ou -OCONH₂.

6. Utilisation selon la revendication 5, dans laquelle R₇ et R₈ représentent chacun un atome d'hydrogène ; R₁ représente un atome de fluor ; et R₃ représente un groupe hydroxyle ou -OCONH₂.

7. Utilisation selon la revendication 1 ou 2 dans laquelle un composé de formule (I) ou un de ses sels est administré ; dans laquelle R₁ représente un atome d'hydrogène, un atome d'halogène, un groupe halogénoalkyle ou hydroxyle ; R₇ et R₈ représentent chacun, indépendamment l'un de l'autre, un atome d'hydrogène ; et R₃ représente un groupe hydroxyle ou -OCONH₂.

8. Utilisation selon la revendication 7 dans laquelle R₁ représente un atome d'hydrogène ; et R₃ représente -OCONH₂.

9. Utilisation selon la revendication 1 ou 2 dans laquelle un composé de formule (III) ou un de ses sels pharmaceutiquement acceptables est administré ; dans laquelle R₁ et R₇ représentent chacun, indépendamment l'un de l'autre, un atome d'hydrogène, un atome d'halogène, un groupe halogénoalkyle et hydroxyle ; R₈ représente un atome d'hydrogène ; et R₄ représente un groupe hydroxyle ou carbonyle.

10. Utilisation selon la revendication 9 dans laquelle R₇ représente un atome d'hydrogène ;

11. Utilisation selon la revendication 7 dans laquelle R₁ représente un atome d'hydrogène ou de fluor ;

12. Utilisation selon la revendication 10 dans laquelle R₁ représente un atome d'hydrogène ou de fluor.

13. Utilisation d'un composé de formule (I), (II) ou (III), ou d'un de ses sels acceptables sur le plan pharmaceutique, tel que défini dans l'une quelconque des revendications 1 à 12, pour la préparation d'un médicament destiné au traitement de l'obésité.

14. Utilisation selon l'une quelconque des revendications 1, 2 ou 13, dans laquelle le médicament est sous forme de dose unitaire qui comprend environ 0,1 mg/kg à environ 1 g/kg du composé.

15. Utilisation d'un composé de formule (I), (II) ou (III), ou d'un de ses sels acceptables sur le plan pharmaceutique, tel que défini dans l'une quelconque des revendications 1 à 12, pour la préparation d'un médicament pour le traitement d'un glaucome.

16. Utilisation selon la revendication 15, dans laquelle le médicament est sous forme de dose unitaire comprenant environ 0,1 mg/kg à environ 1 g/kg dudit composé.

17. Utilisation d'un composé de formule (I), (II) ou (III), ou d'un de ses sels acceptables sur le plan pharmaceutique, tel que défini dans l'une quelconque des revendications 1 à 12, pour la préparation d'un médicament pour le traitement d'un état dépressif.

18. Utilisation d'un composé de formule (I), (II) ou (III), ou d'un de ses sels acceptables sur le plan pharmaceutique, tel que défini dans l'une quelconque des revendications 1 à 12, pour la préparation d'un médicament pour le traitement de troubles de l'humeur.

19. Utilisation d'un composé de formule (I), (II) ou (III), ou d'un de ses sels acceptables sur le plan pharmaceutique, tel que défini dans l'une quelconque des revendications 1 à 12, pour la préparation d'un médicament pour le traitement de la neuropathie diabétique.

20. Utilisation d'un composé de formule (I), (II) ou (III), ou d'un de ses sels acceptables sur le plan pharmaceutique, tel que défini dans l'une quelconque des revendications 1 à 12, pour la préparation d'un médicament destiné au traitement de la maladie d'Alzheimer, de la maladie de Parkinson ou de la démence liée au Sida.

21. Utilisation d'un composé de formule (I), (II) ou (III), ou d'un de ses sels acceptable sur le plan pharmaceutique, tel que défini dans l'une quelconque des revendications 1 à 12, pour la préparation d'un médicament pour le traitement de la méningite, d'une angéite liée au système nerveux central, de la leucodystrophie avec insuffisance surrénale, de l'impuissance, de la schizophrénie, de la toxicomanie, de la sclérose en plaques, de la fatigue, du saturnisme, des myopathies congénitales mitochondriales, de la démence liée au Sida, de la sclérose latérale amyotrophique, du trouble de l'attention, de la narcolepsie, des complications lors d'un accouchement, de l'anesthésie chirurgicale, des lésions dues à un traumatisme crânien et de la moelle épinière, de l'hypoglycémie, du syndrome de Tourette ou de l'encéphalopathie hépatique.
